# EUROPEAN PATENT SPECIFICATION

(11) **EP 3 728 382 B1**
(45) Date of publication and mention of the grant of the patent: **08.12.2021**
(21) Application number: 18822371.3
(22) Date of filing: 20.12.2018
(51) Int. Cl.: C08G 18/73, C08G 18/75, C08G 18/76, C08G 18/80, C08G 18/10, C08G 18/32, C08G 18/28, C09D 7/43

(54) **UREA AND URETHANE GROUP CONTAINING ANTI-SETTLING RHEOLOGY CONTROL ADDITIVE**
HARNSTOFF- UND URETHANGRUPPENHALTIGES RHEOLOGIEKONTROLLADDITIV MIT ANTIABSETZEIGENSCHAFT
GROUPE URÉE ET URÉTHANE CONTENANT UN ADDITIF DE CONTRÔLE RHÉOLOGIQUE ANTI-SÉDIMENTATION

(30) Priority: 21.12.2017 EP 17209556
(43) Date of publication of application: 28.10.2020
(73) Proprietor: BYK-Chemie GmbH, 46483 Wesel (DE)
(72) Inventor: KNAPPKE-BONGARTZ, Christiane, 46483 Wesel (DE); NAGELSDIEK, René, 46483 Wesel (DE); BÜHNE, Sylvia, 46483 Wesel (DE); VON HAAREN, Jan, 46483 Wesel (DE); KLEIN, Agnetha, 46483 Wesel (DE); VERLINDEN, Christoph, 46483 Wesel (DE)
(74) Representative: Altana IP Department
(86) International application number: PCT/EP2018/086342
(87) International publication number: WO 2019/122213

(56) References cited:
- EP-A1- 1 188 779
- WO-A1-2017/017036

## Description

The invention relates to urea and urethane group containing products, their preparation, and their use as rheology control agents, particularly preferred as anti-settling agents. Additionally, the invention relates to rheology control agents (herein also denoted as "rheology agents" or "rheology additives") comprising urea and urethane group containing products and to their use. The invention further relates to liquid compositions and formulations comprising the urea and urethane group containing products.

The rheology of liquid systems is controlled using primarily organically modified bentonites, silicas, hydrogenated castor oil, and polyamide waxes. These substances are mostly dry solids, which must be processed to a semi-finished form using solvents and shearing forces, and/or introduced into the liquid system by means of targeted temperature control. If these temperatures are not observed, crystallites occur in the finished system, and can lead not only to poor rheological performance, but also to detrimental properties of the products.

In case the liquid systems are coatings, these rheological auxiliaries frequently lead to instances of clouding and haze in clear, transparent coatings. Moreover, operating with dry, powderous products, which cause dusts during processing, may be technologically unfavorable.

Other solutions for rheology control have been set out in European patent application EP-A-0198519. Here, an isocyanate is reacted with an amine, in the presence of solutions of film-forming resin, to form a urea, which forms microcrystalline, needle-shaped crystals. These film-forming binders, thus modified, are used as rheology control binders and sag-preventing binders, in the form of what are called "sag control agents".

Other proposals for rheology control are described in US 4,311,622 and US 4,677,028, where polyisocyanates or polyisocyanurates are reacted with monoamines or polyamines in the mandatory presence of a binder, to form polyureas.

WO 02/04579 describes ureas which are used for thickening fats or oils. These thickeners are prepared by stoichiometric reaction of primary amines with diisocyanates in the fat or oil which is to be thickened.

Patent specification US 5,554,586 likewise describes the thickening of oils *in situ.* In this case, a mixture of primary monofunctional amines with polyoxyalkylene diamines is reacted with diisocyanates in the oil to be thickened.

US 2005/0182205 and WO 95/09201 both describe the thickening of molding compounds (bulk molding compounds, BMC, and sheet molding compounds, SMC) using urea derivatives that are obtained by reacting isocyanates with diamines or triamines. As the isocyanate component, it is possible to use aliphatic or aromatic diisocyanates, but also reaction products of diisocyanates with polyetherdiols or polyesterdiols. As the amine component, low molecular weight diamines and triamines, and polyamines, are employed. The urea compounds are prepared by mixing the amine component and isocyanate component in the corresponding resin.

The disadvantage of most of the products described in the above prior art is that they always should be prepared in the medium which is to be thickened, and whose rheology they are supposed to influence. The products, therefore, are not independent of the medium to be thickened. They are not stable on storage, but instead exhibit lumps and/or bits after a short time. A further disadvantage is that these thixotroped media often must be prepared with the aid of a pre-gel. This viscous pre-gel must typically be processed immediately after its preparation, since after a prolonged standing time it can no longer be incorporated without disruption. Subsequent correction of completed formulations is therefore not possible. Most of the rheology control agents of the prior art cannot be prepared alone, but only in the presence of other components being part of the liquid formulation that needs to be thickened, e.g., of film-forming agents. Their usefulness is therefore limited.

EP 1188779 describes a process for preparing a solution which is effective as a thixotropic agent and comprises urea-urethanes, and use of this solution for the thickening of coating materials. These urea-urethanes are obtained by reacting monohydroxy compounds with an excess of tolylene diisocyanate, removing the unreacted portion of the tolylene diisocyanate from the reaction mixture, and further reacting the resulting monoisocyanate adducts with diamines in a molar ratio of 2:1, in a solvent, to form urea-urethanes. EP-A-0006252 describes analogous urea-urethanes, which are obtained by stoichiometric reaction of monohydroxy compounds with diisocyanates and diamines.

Patent specification DE 10241853 B3 describes polymeric urea-urethanes obtainable by a first reaction of an excess of diisocyanate with a polyol, to form a double-sidedly NCO-terminated urethane polymer, present alongside excess diisocyanate, and subsequent second reaction of the mixture of the double-sidedly NCO-terminated urethane prepolymer and the excess diisocyanate, on the one hand, and a mixture of a primary monoamine and a primary diamine, on the other. Reaction media used are polar aprotic solvents. The urea-urethane solutions obtained in this way are used as rheology control agents in liquid polymer systems. The disadvantage of these urea-urethanes is the limited shear stability, and the thixotropy.

Further important application areas where rheology control agents are used are gas and oil well drilling fluids, such as for example described in WO 02/42392. Such oil based fluids often contain hydrophilic polymers in particulate form as environmentally acceptable thickeners for use in onshore and offshore drilling. The particulate hydrophilic polymers can have a high solids content in the oil-based fluid carrier. Many hydrophilic polymers used in such applications are selected from the group of polysaccharides such as cellulose ethers, guar gum and its derivatives and starch and its derivatives. The cellulose ethers particularly include carboxymethyl cellulose (CMC), hydroxyethyl cellulose (HEC), carboxymethyl hydroxyethyl cellulose (CMHEC), polyanionic cellulose (PAC); guar gum and guar gum derivatives including straight guar (Guar), carboxymethyl guar (CMG), hydroxypropyl guar (HPG), and carboxymethyl hydroxyethyl guar (CMHPG); and starch including carboxymethyl starch, hydroxyethyl starch, and hydroxypropyl starch. Guar and its derivatives are the most extensively used polymers in gas and oil well drilling fluids. Guar is used to thicken the fluid so that it can carry graded sand (proppant) into the geological formation. Polysaccharides like guar and its derivatives can also be used as viscosifiers and fluid loss additives in low solids drilling muds. Due to their non-ionic nature and high mean average molecular weight, guar can develop viscosity in water or brines. However, the polysaccharide particles tend to settle in the oil based fluids on storage, the oil based fluids being used to transport the particles as part of a pumpable liquid formulation to the place where the thickening of an aqueous medium is intended.

WO 2017/017036 relates to linear urea urethanes as rheology control agents, which contain at least one terminal residue selected from a mono- or polyunsaturated, branched or unbranched alkenyl or alkynyl radical having 12 to 24 carbon atoms. However, those rheology control agents still have potential for improvement, if to be used in the field of gas and oil production.

It was an object of the present invention, therefore, to provide new rheology control agents. These new agents ought not to have the disadvantages stated in the abovementioned specifications. More particularly the intention was to find rheology control agents, which may be provided in organic formulations for use in water-based fluids for oil and gas drilling, completion and production purposes. They ought to have an excellent compatibility with fluids used in gas and oil production and should allow for an improved anti-settling property of particulate ingredients of the formulation, particularly particulate polysaccharides used for thickening purposes, as for example Guar Gum or the before-mentioned cellulose ether derivatives.

The rheology control agents ought additionally to be useful as general purpose anti-settling agents for increasing the storage stability. In systems comprising low or medium polar solvents such as mineral oils, paraffin oils, less polar alcohols or esters, the rheology control agents ought to exhibit good compatibility and a good rheological activity.

Surprisingly it has been found that these objectives can be achieved by providing a urea and urethane group containing product comprising one or more species of formula (I)

R¹-O-(C=O)-NH-R²-NH-(C=O)-NH-R³-NH-[-(C=O)-NH-R⁴-NH-(C=O)-NH-R³-NH-]ₙ-(C=O)-NH-R²-NH-(C=O)-O-R¹ (I)

, wherein
R¹ independently represent non-aromatic hydrocarbyl groups having 14 to 30 carbon atoms;
R² independently represent alkyl-substituted aromatic hydrocarbyl groups having 7 to 12 carbon atoms;
R³ independently represent hydrocarbyl groups having 2 to 36 carbon atoms, which can be interrupted by 1 to 17, preferably 1 to 10, more preferably 1 to 5, most preferably 1, 2 or 3 ether oxygen atoms in case of aliphatic hydrocarbyl groups;
R⁴ independently represent hydrocarbyl groups having 2 to 36 carbon atoms;
n is an integer from 0 to 200, preferably 0 to 150, more preferred 0 to 100 and most preferred 0 to 50 carbon atoms; and
wherein on average from 40 mol-% to 100 mol-% of all R³ and R⁴ groups contained in the one or more species of formula (I) are acyclic aliphatic hydrocarbyl groups, which, in case of R³, can be interrupted by 1 to 17 ether oxygen atoms.

The term "urea and urethane group containing product" means any product, particularly any reaction product containing one or more of species of formula (I) defined as above. The average of 40 mol-% to 100 mol-% of all R³ and R⁴ groups is calculated on the total number of moles of R³ and R⁴ groups contained in the urea and urethane group containing product of formula (I). Therefore, the proviso that on average from 40 mol-% to 100 mol-% of all R³ and R⁴ groups contained in the one or more species of formula (I) are acyclic aliphatic hydrocarbyl groups, which, in case of R³, can be interrupted by 1 to 17 ether oxygen atoms, is satisfied even if the urea and urethane group containing product contains single species wherein no residue R³ and/or R⁴ is an acyclic aliphatic hydrocarbyl group, which, in case of R³, can be interrupted by 1 to 17 ether oxygen atoms, are contained if the collective of all species of formula (I) satisfies this requirement on average.

The term "hydrocarbyl groups" denotes for an organic group, which consists of carbon and hydrogen atoms, only. A "hydrocarbyl group", which may be interrupted by 1 or more ether oxygen atoms, is e. g., a group of formulae CH₂-CH₂-O-CH₂-CH₂ (interrupted by 1 ether oxygen atom) or CH₂-CH₂-O-CH₂-CH₂-O-CH₂-CH₂ (interrupted by 2 ether oxygen atoms).

The term "aliphatic group" as used herein refers to a radical of an acyclic or cyclic, saturated or unsaturated carbon compound that does not contain aromatic structures (see: IUPAC Compendium of Chemical Terminology, 2nd Ed. (The "Gold Book") A. D. McNaught and A.

Wilkinson, Blackwell Scientific Publications, Oxford (1997) XML online corrected version: http://goldbook.iupac.org (2006-) created by M. Nic, J. Jirat, B. Kosata; updates compiled by A. Jenkins, ISBN 0-9678550-9-8, https://doi.org/10.1351/goldbook). Accordingly, aliphatic groups or radicals may contain heteroatoms such as, for example, oxygen or nitrogen. As an example oxygen can be present in an aliphatic group in form of ether and/or ester groups. E.g. a polyoxyalkylene group is a heteroatom (in this case oxygen) containing aliphatic group. Aliphatic groups can also contain aliphatic and aromatic moieties at the same time. E.g. an aliphatic group which contains one or more aromatic groups as substituents is called araliphatic group. According to the well-established nomenclature any aliphatic group containing cycloaliphatic moieties and no aromatic moieties, is a cycloaliphatic group.

### Species of formula (I)

### R¹ groups

Groups R¹ are independently selected from non-aromatic hydrocarbyl groups having 14 to 30 carbon atoms.

Preferably R¹ is an acyclic aliphatic group having 14 to 30, more preferably 16 to 28 and most preferred 18 to 26 or even 18 to 22 carbon atoms. Such aliphatic group R¹ can be saturated or unsaturated, branched or linear. More preferably R¹ is branched and/or unsaturated. Most preferable R¹ is an ethylenically unsaturated hydrocarbyl group, as particularly preferred an oleyl group or R¹ is a saturated branched hydrocarbyl group. In case of an ethylenically unsaturated hydrocarbyl group it is most preferred if the carbon-carbon double bond of the ethylenically unsaturated group has cis-geometry, as e. g. realized in an oleyl group.

### R² groups

Groups R² are independently selected from alkyl-substituted aromatic hydrocarbyl groups having 7 to 12 carbon atoms. The R² groups are divalent groups since they are only bound to the adjacent NH groups of the species of formula (I). Preferred are divalent benzene residues, having one or more alkyl groups bound to the benzene ring as substituents, the alkyl group or alkyl groups preferably containing 1 to 4, more preferably 1 or 2 carbon atoms and most preferred being methyl groups.

Preferred groups R² are toluylene groups, and particularly 2,4-toluylene groups and 2,6-toluylene groups and mixtures thereof.

### R³ and R⁴ groups

Groups R³ and R⁴ are independently selected from hydrocarbyl groups having 2 to 36 carbon atoms. In addition, aliphatic R³ hydrocarbyl groups, particularly acyclic aliphatic R³ hydrocarbyl groups can be interrupted by 1 to 17, preferably 1 to 10, more preferably 1 to 5, most preferably 1, 2 or 3 ether oxygen atoms. However, it is preferred that aliphatic R³ hydrocarbyl groups are not interrupted by any ether oxygen atoms, i.e., R³ consists of carbon and hydrogen atoms only.

Groups R³ and R⁴ can be aromatic or aliphatic. Preferably groups R³ and R⁴ are aliphatic, most preferably groups R³ and R⁴ are acyclic. An acyclic aliphatic hydrocarbyl groups does not contain cyclic moieties such as cyclohexylene moieties or aromatic moieties.

Groups R³ are independently selected from hydrocarbyl groups having 2 to 36 carbon atoms, preferably 2 to 20 carbon atoms, more preferred 2 to 12, most preferably 2 to 8 or even 2 to 6 carbon atoms, whereby those groups can be interrupted by 1 to 17, more preferably 1 to 10, even more preferably 1 to 5, most preferably 1, 2 or 3 ether oxygen atoms. However, it is preferred that aliphatic R³ hydrocarbyl groups are not interrupted by any ether oxygen atoms. The R³ groups are divalent groups since they are only bound to the adjacent NH groups of the species in formula (I).

Examples of suitable R³ groups are -(CH₂)ₚ- or -[CH₂CH₂O]_{(0.5*p)}CH₂CH₂-, with p = 2 to 20, preferably p = 2 to 16 more preferably p = 2 to 12, -CH₂CH(CH₃)-, -CH₂C(CH₃)₂CH₂-, the divalent radical of 3-methyl-3,5,5-trimethylcyclohexane, with radical positions at the 1-position of the cyclohexane ring and the methyl group at the 3-position of the cyclohexane ring, the divalent radicals of cyclohexane, dicyclohexylmethane, 3,3'-dimethyl-dicyclohexylmethane, the para- and meta-xylylene radicals, the divalent radicals of diphenylmethane, 3,3-dimethyl-diphenylmethane and benzene. In a typical embodiment, R³ is selected from -CH₂CH₂-, -CH₂CH₂CH₂-, -CH₂CH₂CH₂CH₂-, and -CH₂CH₂CH₂CH₂CH₂CH₂-, preferably selected from -CH₂CH₂CH₂- and -CH₂CH₂CH₂CH₂-.

Groups R⁴ are independently selected from hydrocarbyl groups having 2 to 36 carbon atoms. Preferably groups R⁴ are independently selected from hydrocarbyl groups having 4 to 24 carbon atoms, more preferably 5 to 18 or even 6 to 13 carbon atoms. The hydrocarbyl groups R⁴ are aromatic or aliphatic.

In case of aliphatic hydrocarbyl groups R⁴, acyclic aliphatic hydrocarbyl groups are preferred. The R⁴ groups are divalent groups since they are only bound to the adjacent NH groups of the species of formula (I). R⁴ groups can be the same as R² groups if the proviso that on average at least 40 mol-% of all groups R³ and R⁴ present in the species according to formula (I) are acyclic aliphatic hydrocarbyl groups is fulfilled. Preferably R⁴ is an acyclic, aliphatic hydrocarbyl group. It is therefore very much preferred that the groups R² and R⁴ in the species of formula (I) are different from each other.

Examples of groups R⁴ are *CH₂-CH₂-CH₂-CH₂-CH₂*, *CH₂-CH₂-CH₂-CH₂-CH₂-CH₂*, *CH₂-C(CH₃)₂-CH₂-CH(CH₃)-CH₂-CH₂*, *CH₂-CH(CH₃)-CH₂-C(CH₃)₂-CH₂-CH₂*, the hydrocarbon moiety of a dimer diisocyanate based on the hydrocarbon backbone of a dimerized fatty acid, or any of the following groups 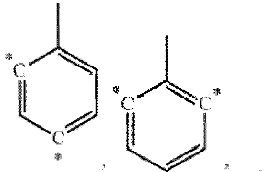 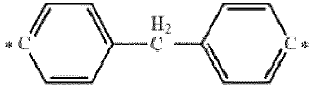 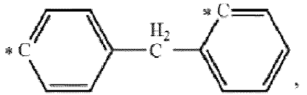 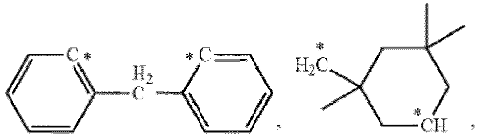 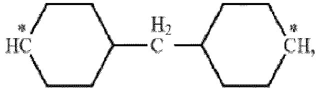 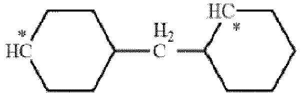 and 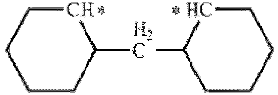 wherein the asterisk symbol * denotes the positions where R⁴ is bound to the adjacent NH groups in formula (I). From the above groups the preferred groups are the *CH₂-CH₂-CH₂-CH₂-CH₂*, *CH₂-CH₂-CH₂-CH₂-CH₂-CH₂*, *CH₂-CH(CH₃)-CH₂-C(CH₃)₂-CH₂-CH₂* and *CH₂-C(CH₃)₂-CH₂-CH(CH₃)-CH₂-CH₂* groups, the hexamethylene group being most preferred.

To solve the problems addressed in the present invention it is necessary that on average at least 40 mol-%, preferably at least 50 mol-%, more preferably at least 60 mol-%, even more preferably at least 70 mol-% and most preferably at least 80 mol.-% or even at least 90 mol-% up to 100 mol-% of all groups R³ and R⁴ present in the species according to formula (I) are acyclic aliphatic hydrocarbyl groups, which, in case of R³, can be interrupted by 1 to 17 ether oxygen atoms. It is utmost preferred that all groups R³ and R⁴ present in the species according to formula (I) are acyclic aliphatic hydrocarbyl groups, which, in case of R³, can be interrupted by 1 to 17 ether oxygen atoms. In some embodiments, R³ does not contain ether oxygen atoms.

Particularly preferred 90 mol-%, more preferred 95 mol-% and most preferred 100 mol-% of groups R³ are acyclic aliphatic hydrocarbyl groups, which can be interrupted by 1 to 17 ether oxygen atoms. In some embodiments R³ does not contain ether oxygen atoms.

Particularly preferred 90 mol-%, more preferred 95 mol-% and most preferred 100 mol-% of groups R⁴ are acyclic aliphatic hydrocarbyl groups.

The most preferred acyclic aliphatic hydrocarbyl groups R³ and R⁴ are acyclic, linear or branched, saturated hydrocarbyl groups. Particularly preferred are such acyclic, linear or branched, saturated hydrocarbyl having the formula [CR^{a}₂]ₖ with R^{a} independently being H or an alkyl group with 1 to 6, preferably 1 to 4 or even more preferred 1 or 2 carbon atoms, and k being an integer from 2 to 20, preferably 2 to 16, more preferred 2 to 12 or 2 to 8 carbon atoms. In case of R⁴ groups it is particularly preferred that k is an integer of at least 4, more preferred at least 6, while the upper limits of k are the same as above. In case of R³ groups it is particularly preferred that k is an integer of at least 2, while the upper limit of k is 6, preferably 5, more preferably 4, and most preferably 3. Both for R³ and R⁴, it is very much preferred that R^{a} is hydrogen.

### Manufacture of the urea and urethane group containing products of the invention

Briefly, the urea and urethane group containing product of the invention can be obtained by first reacting one or more components R¹-OH with one or more diisocyanates OCN-R²-NCO to form one or more monoisocyanato adducts having the following formula (II)

R¹-O-(CO)-NH-R²-NCO (II),

wherein R¹ and R² are defined as above. This reaction is usually carried out with a molar excess of diisocyanates OCN-R²-NCO to prevent the formation of by-products. The excess of diisocyanates OCN-R²-NCO can be removed, e.g. by distillation, before carrying out the following second step. However, alternatively, it is possible to leave the excessive amount of diisocyanates OCN-R²-NCO in the mixture, if at least some of the diisocyanates OCN-R²-NCO used in the first step are the same as used in the second step. If the diisocyanates OCN-R²-NCO used in the first step are the same as those used in the second step and if the excessive amount used in the first step equals the amount to be used for forming a mixture in the second step, i.e. the crude product obtained in the first step is the same as the mixture to be formed in the second step, the second step can even be skipped and it can directly be proceeded with the third step. However, since it is preferred that R² and R⁴ are different from each other; this method in which the second step can be skipped is the less preferred method of preparation.

In an optional second step the one or more adducts of formula (II) are mixed with one or more diisocyanates OCN-R⁴-NCO, wherein R⁴ is defined as above, to form a mixture.

In a further step the one or more adducts of formula (II) or the mixture of the optional second step is further reacted with one or more diamines H₂N-R³-NH₂, wherein R³ is defined as above to give a urea and urethane group containing product of the invention, containing one or more species of formula (I).

### Stoichiometry

The value of n can be adjusted by the stoichiometry between species of formula (II), diisocyanates OCN-R⁴-NCO and diamines H₂N-R³-NH₂. The species of formula (II) will form the two terminal moieties of the species of formula (I). The higher the number of species of formula (II), the lower the number average weight (Mₙ) and weight average molecular weight (M_{w}) of species of formula (I) will be.

Preferably the number average molecular weight (Mₙ) of the urea and urethane group containing products of the present invention ranges from 1200 to 8000 g/mol, preferably 1500 to 6000 g/mol, more preferably 1800 to 4200 g/mol, even more preferably from 2000 to 4000 g/mol and most preferably from 2200 to 3800 g/mol, determined by gel permeation chromatography (eluent: dimethylacetamide + 5 g/L lithium bromide; column: combination of 3 PSS-PolarSil colums supplied by Polymer Standard Service, dimension 300 mm * 8 mm ID per column, particle size 5 µm, pore size 1*1000 Å, 1*300 Å, 1* 100 Å; temperature: 50 °C; standard: polymethylmethacrylate standards with Mₚ from around 1000000 to 102) according to DIN 55672 part 2 (year: 2008). It was also found that the polydispersity (P_{D}) of the urea and urethane group containing products (M_{w}/Mₙ) is preferably below 1.5, more preferably below 1.4, even more preferably below 1.3 or below 1.2. To determine the polydispersity (P_{D}), the weight average molecular weight of the urea and urethane group containing products was also obtained by the before described gel permeation chromatography method.

### Stabilizers

The reactions may take place in the presence of ionogenic compounds. As ionogenic compounds preferably salts are used containing cations of elements of the main groups I and II of the Periodic Table of the Elements (alkali and alkaline earth metals) or ammonium ions, preferably lithium, calcium or magnesium, particularly preferably lithium and calcium cations, and containing as anions preferably monovalent anions, particularly preferably halides, pseudohalides, formate, acetate and/or nitrate, most particularly preferably chloride, acetate and/or nitrate.

Particularly preferred as ionogenic compounds are soluble inorganic lithium salts, such as lithium chloride or lithium nitrate, for example. When ionic liquids are used as a carrier and/or solvent, it is possible to forego the use of the above stabilizers.

In the context of the present invention so-called ionic liquids (i.e. organic salts with a melting point ≤ 80°C) are not subsumed under the term ionogenic compounds, but rather belong to solvents and/or carrier media.

The amount of ionogenic compound, preferably lithium compound is preferably 0.2 to 2.5, more preferably 0.1 to 1.5 and even more preferably 0.6 to 1.0 times the molar amount of the one or more diamines H₂N-R³-NH₂.

In the processes for preparing the polyureas of the invention it is advantageous to use lithium compounds or liquid salts, to increase the storage stability of the rheology control agent systems.

### Solvents

The reaction is usually carried out in an aprotic polar organic solvent. Suitable solvents are selected from the group of amides, preferably cyclic amides (i. e. lactams), sulfoxides, preferably dimethyl sulfoxide and/or ionic liquids. Further suitable aprotic solvents which can be used in the manufacture of the urea group containing products of the invention are listed in the section on liquid compositions as suitable carrier media for the rheology control agents. Particularly suitable are solvents selected from the group of N-alkyl-lactams, preferable N-alkyl butyrolactams and even more preferred N-C₁₋₈-alkyl-butyrolactams, like N-butyl-butyrolactam. The solvents used for synthesis can also be used as carrier media of the liquid compositions of the invention.

### Reaction temperature and time

The choice of the respective reaction conditions, as e. g. the reaction temperature, reaction time and dosing rates are known to the skilled person and are illustrated in more detail in the working examples.

### Reactants

### Component R¹-OH

Suitable components R¹-OH are those, wherein R¹ is defined as above.

Specific examples of components R¹-OH are saturated or unsaturated, linear or branched aliphatic hydrocarbyl monoalcohols having 14 to 30 carbon atoms, preferably having 16 to 28 carbon atoms.

Examples of such monoalcohols are Guerbet alcohols with a chain length of C₁₄ to C₂₀, fatty alcohols, such as oleyl alcohol, linoleyl alcohol, palmityl alcohol, stearyl alcohol or the alkyl-substituted derivatives thereof.

It is particularly preferred that alcohols R¹-OH are liquid at 23 °C and standard pressure (100 kPa). Since many of the ethylenically unsaturated alcohols of formula R¹-OH with 14 to 30 carbon atoms in R¹ are liquid, this requirement is best fulfilled in case R¹ is an ethylenically unsaturated aliphatic hydrocarbyl group or a branched saturated aliphatic hydrocarbyl group having 14 to 30, preferably 16 to 28 and even more preferred 18 to 26 carbon atoms. It is also possible to use mixtures of two or more alcohols R¹-OH, where it is preferred that such mixture is liquid at 23 °C and standard pressure (100 kPa). Such liquid mixtures can contain or consist of alcohols R¹-OH which themselves are not liquid at 23 °C and standard pressure.

### Diisocyanates OCN-R²-NCO and OCN-R⁴-NCO

Suitable diisocyanates OCN-R²-NCO and OCN-R⁴-NCO are those, wherein R² and R⁴, respectively, are defined as above.

Preferred diisocyanates OCN-R²-NCO are 2,6-toluene diisocyanate, 2,4-toluene diisocyanate and mixtures thereof.

Preferred diisocyanates OCN-R⁴-OCN are 1,4-tetramethylene diisocyanate, 1,5-pentamethylene diisocyanate, 1,6-hexamethylene diisocyanate, 2,2,4-trimethyl-1,6-hexamethylene diisocyanate, 2,4,4-trimethyl-1,6-hexamethylene diisocyanate, 1,10-decamethylene diisocyanate, 1,4-cyclohexane diisocyanate, isophorone diisocyanate, p-phenylene diisocyanate, m-phenylene diisocyanate, 2,6-toluene diisocyanate, 2,4-toluene diisocyanate and mixtures thereof, p- and m-xylylene diisocyanate, 4,4'-diisocyanatodicyclohexylmethane, 3,3'-dimethyl-4,4'-bisphenylene diisocyanate, 3,3'-dimethyl-diisocyanatodicyclohexylmethane, the isomer mixtures of 2,4'- and 4,4'-diisocyanatodiphenylmethane, and C₃₆ dimer diisocyanate; and most preferred diisocyanates OCN-R⁴-NCO are - since they contribute to the fulfillment of the proviso that on average at least 40 mol-% of groups R³ and R⁴ acyclic aliphatic hydrocarbyl groups - 1,4-tetramethylene diisocyanate, 1,5-pentamethylene diisocyanate, 1,6-hexamethylene diisocyanate (HDI), 2,2,4-trimethyl-1,6-hexamethylene diisocyanate, 2,4,4-trimethyl-1,6-hexamethylene diisocyanate, 1,10-decamethylene diisocyanate, and acyclic C₃₆ dimer diisocyanates, under which HDI is particularly preferred.

### Diamines H₂N-R³-NH₂

Suitable diamines H₂N-R³-NH₂ are those, wherein R³ is defined as above.

Specific examples of such diamines are e.g. acyclic aliphatic diamines as ethylenediamine, neopentanediamine, 1,2- and 1,3-propanediamine, 1,4-butanediamine, 1,5-pentanediamine,1,6-hexamethylenediamine, 1,8-octamethylenediamine, 1,10-decamethylenediamime, 1,12-dodecamethylenediamine; cycloaliphatic diamines as cyclohexyldiamine, 4,4'-diaminodicyclohexylmethane, 3,3'-dimethyl-4,4'-diaminodicyclohexylmethane, isophorone-diamine; and araliphatic diamines like para- and meta-xylylenediamine or isomeric xylylenediamines; and aromatic diamines like 4,4'-diaminodiphenylmethane, 3,3'-dimethyl-4,4'-diaminodiphenylmethane and isomers of phenylenediamine.

Since R³ can be a hydrocarbyl group which is interrupted by 1 to 17, preferably 1 to 10, more preferably 1 to 5, most preferably 1, 2 or 3 ether oxygen atoms, polyether diamines such as H₂N-CH₂-CH₂-O-CH₂-CH₂-O-CH₂-CH₂-NH₂ (Jeffamine^{®} EDR 148) can also be used.

Amongst the before-mentioned diamines the acyclic aliphatic diamines are preferred, since they contribute to the fulfillment of the proviso that on average at least 40 mol-% of groups R³ and R⁴ must be acyclic aliphatic hydrocarbyl groups. In one embodiment, R³ does not contain ether oxygen atoms.

### Liquid Compositions comprising the urea and urethane group containing product of the invention

The term "liquid composition" according to the present invention denotes a composition, i. e. a matter of at least two substances, being liquid, i. e. flowable at 23 °C and 100 kPa, wherein one of the at least two substances is the urea and urethane group containing product of the invention.

The term liquid composition as used herein also includes semi-finished products and final products, which themselves contain the urea and urethane group containing product of the invention or the rheology control agent comprising at least one urea and urethane group containing product of the invention, and preferably a carrier medium and a further ingredient which is different from the at least one urea group containing product of the invention and the carrier medium. An example for a semi-finished product is a fluid or slurry for gas and oil production or metal working. Such a slurry may contain inorganic and/or organic particles such as polysaccharides and their derivatives or baryte. The purposes of the particle can be manifold, e.g., they can be used as rheology additives (preferably thickeners which become active in contact with water), weighting agents, proppants (e.g., as lightweight porous materials), gas generating additives, such as metal particles (e.g., aluminum particles under alkaline conditions), or lubricating aid/lubricating additives.

In the simplest case the liquid composition consists of the urea and urethane group containing product of the invention and a carrier medium. The carrier medium can be the solvent or mixture of solvents wherein the manufacture of the urea and urethane group containing product of the present invention was carried out. In such case, the liquid composition is preferably substantially clear to hazy, preferably has low- to medium-viscosity, forms a solution or dispersion having preferred active ingredient fractions, i. e. fractions of the urea and urethane group containing product of the invention from 10 to 70 % by weight, more preferably 15 to 55% by weight, and most preferably 20 to 50% by weight, based on the total weight of the liquid composition. Such simple liquid compositions, e.g. serve as rheology control agents and are rheology control agents according to the present invention.

The liquid composition can e. g. be used as a rheology control agent which comprises at least one urea and urethane group containing product of the invention. Examples of carrier media are organic solvents, which may be polar or non-polar. The urea and urethane group containing product may be present, for example, in solution or dispersion in the carrier medium. The rheology control agent itself may take the form of a solution, dispersion such as emulsion or suspension, gel or paste. Where the rheology control agent is to be in the form of a solution, it is preferred to use polar aprotic solvents. A preferred carrier medium for pastes comprises, suitably, non-polar solvents such as paraffinic hydrocarbons and mineral oils, preferably having a low content of aromatic compounds, or being free of aromatic compounds (e.g., so-called BTEX free mineral oils). It is, however, very much preferred that the rheology control agent is delivered in the form of a solution.

Preferably, the rheology control agents according to the invention are present as a solution in aprotic organic solvents. Particularly suitable are polar, aprotic organic solvents, very particularly those which are selected from the group consisting of linear amides (including etheramides and esteramides), lactams, sulfoxides and ionic liquids (i.e. organic salts with a melting point ≤ 80°C). It is therefore preferred to use such solvents as carrier medium and/or to carry out the preparation of the inventive rheology control agents in these polar, aprotic organic solvents or ionic liquids.

Such a liquid composition preferably comprises or consists of
(a) 5 to 70 % by weight of one or more urea group containing products according to the invention,
(b) 30 to 95 % by weight of one or more polar aprotic solvents and/or ionic liquids, and
(c) 0 to 8 % by weight of one or more ionogenic compounds,
   the amounts of (a), (b) and (c) being based on the total weight of the liquid composition.

More preferred, such a liquid composition comprises or consists of
(a) 10.0 to 55.0 % by weight of one or more urea group containing products according to the invention,
(b) 44.8 to 89.8 % by weight of one or more polar aprotic solvents and/or ionic liquids, and
(c) 0.2 to 6.0 % by weight of one or more ionogenic compounds,
   the amounts of (a), (b) and (c) being based on the total weight of the liquid composition.

Even more preferred, such a liquid composition comprises or consists of
(a) 15.0 to 50.0 % by weight of one or more urea group containing products according to the invention,
(b) 49.5 to 84.5 % by weight of one or more polar aprotic solvents and/or ionic liquids, and
(c) 0.5 to 5.0 % by weight of one or more ionogenic compounds,
   the amounts of (a), (b) and (c) being based on the total weight of the liquid composition.

Most preferred, such a liquid composition comprises or consists of
(a) 20.0 to 45.0 % by weight of one or more urea group containing products according to the invention,
(b) 54.0 to 79.0 % by weight of one or more polar aprotic solvents and/or ionic liquids, and
(c) 1.0 to 4.0 % by weight of one or more ionogenic compounds,
   the amounts of (a), (b) and (c) being based on the total weight of the liquid composition.

Particularly preferred polar aprotic organic solvents are substituted or unsubstituted, preferably unsubstituted N-alkylbutyrolactams, dialkyl sulfoxides, substituted or unsubstituted amides, especially carboxamides. Examples of N-alkylbutyrolactams are N-methylbutyrolactam, N-ethylbutyrolactam, N-butylbutyrolactam, N-octylbutyrolactam, N-decylbutyrolactam, N-dodecylbutyrolactam, and N-hydroxyethyl butyrolactam. An example of a dialkyl sulfoxide is dimethyl sulfoxide. Examples of linear amides are N,N-dimethylformamide, N,N-dimethylacetamide, N,N-dimethyloctanamide, N,N-dimethyldecanamide, N,N-dimethyldodecanamide, 2-hydroxy-N,N-dimethyl-propanamide, N,N-dialkylamidoalkyl esters, N,N-dialkylamidoalkyl ethers, hexamethylphosphoric acid triamide and acylmorpholines. Preferred ionic liquids suitable as solvents are substituted imidazolium salts, e.g. 1-ethyl-3-methylimidazolium acetate, 1-ethyl-3-methylimidazolium acetate, 1-ethyl-3-methylimidazolium ethylsulfate, 1-butyl-3-methylimidazolium ethylsulfate, 1-ethyl-3-methylimidazolium thiocyanate and 1-butyl-3-methylimidazolium thiocyanate. The solvents and ionic liquids can also be used in combinations.

Among the solvents, preference is given to dimethylsulfoxide and, in particular, to such N-alkylbutyrolactams whose nitrogen-bonded alkyl radical is linear or branched, preferably linear, and the alkyl radical contains 1 to 20 or preferably 1 to 16, more preferably 1 to 12 and most preferably 3 to 10 carbon atoms, N,N-dimethylamides of C₃ to C₁₂ carboxylic acids, and also N,N-dimethylamidoalkyl esters (e.g., methyl 5-(dimethylamino)-2-methyl-5-oxopentanoate), N,N-dimethylamidoalkyl ethers (e.g., 3-methoxy-N,N-dimethylpropionamide), formylmorpholine and acetylmorpholine.

Depending on the application, those solvents are particularly preferred which have a corresponding miscibility with water, e.g. N-methylbutyrolactam, N-ethylbutyrolactam, N-propylbutyrolactam, N-butyl-butyrolactam, and dimethyl sulfoxide.

To enhance the solubilizing properties of the solvent or solvents used in liquid compositions, particularly liquid rheology control agents, ionogenic compounds can be used. As ionogenic compounds preferably salts are used containing cations of elements of the main groups I and II of the Periodic Table of the Elements (alkali and alkaline earth metals) or ammonium ions, preferably lithium, calcium or magnesium, particularly preferably lithium and calcium cations, and containing as anions preferably monovalent anions, particularly preferably halides, pseudohalides, formate, acetate and/ or nitrate, most particularly preferably chloride, acetate and/ or nitrate. The lithium salts are very much preferred among those.

The rheology control agents which comprise at least one urea and urethane group containing product of the invention and preferably a carrier medium, can be easily incorporated e. g. into hydrocarbon based slurries used in gas and oilfield completion, metal working fluids, paints and polymeric systems, with no need for extensive shearing. Working with liquid compositions has the further advantages that they can be processed in dust-free form, are substantially transparent, exhibit particularly good compatibility with other systems, for example. They allow effective anti-settling via a yield point without extremely increasing the viscosity.

Amongst the oils to which the species of formula (I) and/or the rheology control agents containing such species may be added are such oils, which are used in the gas and oil production. Such oils are used in all stages of gas and oil production, including drilling, completion and production. Preferably the species of formula (I) and/or the rheology control agents containing such species may be added to such oils that are used during completion. Particularly preferred are such fluids further containing organic or inorganic particles. Preferred inorganic particles are those containing or consisting of graphite, graphene, silica and silicates (e.g., sand or glass beads), molybdenum disulfide, baryte, silicon carbide, silicon nitride, oxycarbides and oxynitrides of silicon, metal flakes (e.g., aluminum, copper, zinc, silver, gold and their alloys), and ceramic materials. Among the organic particles, hydrophilic polymer particles are preferred, most preferably particles containing or consisting of polysaccharides or their derivatives. In another preferred embodiment, hydrophobic particles (e.g., PTFE = polytetrafluorethylene) can be used as organic particles.

Therefore, a further important application area where the species of formula (I) and/or the rheology control agents of the present invention are used are gas and oil well drilling fluids, such as for example described in WO 02/42392. Particularly species of formula (I) and/or rheology control additives of the present invention can be successfully used in oil based fluids containing particles, preferably organic particles, especially hydrophilic polymers in particulate form for use in onshore and offshore drilling, stimulation, completion, and production. The particles, preferably organic particles, especially particulate hydrophilic polymers can have high solids content in the oil-based fluid carrier. Many hydrophilic polymers used in such applications are selected from the group of polysaccharides such as cellulose ethers, guar gum and its derivatives, xanthan gum and its derivatives and starch and its derivatives. The cellulose ethers particularly include carboxymethyl cellulose (CMC), hydroxyethyl cellulose (HEC), carboxymethyl hydroxyethyl cellulose (CMHEC), polyanionic cellulose (PAC); guar gum and guar gum derivatives including straight guar (Guar), carboxymethyl guar (CMG), hydroxypropyl guar (HPG), and carboxymethyl hydroxyethyl guar (CMHPG); and starch including carboxymethyl starch, hydroxyethyl starch, and hydroxypropyl starch. Guar and its derivatives are the most extensively used polymers in gas and oil well drilling fluids. Guar is for example used to thicken the fluid so that it can carry graded sand (proppant) into the geological formation. Polysaccharides like guar and its derivatives can also be used as viscosifiers and fluid loss additives in low solids drilling muds. Due to their non-ionic nature and high mean average molecular weight, guar can develop viscosity in water or brines. The species according to formula (I) and/or the rheology additives of the present invention can successfully prevent settling of hydrophilic polymers in particulate form in such oil based fluids on storage. It is therefore very beneficial to prepare a slurry of the aforementioned particles in a fluid carrier, preferably a hydrocarbon, to get a storage stable slurry, i.e., a slurry that is stabilized against settling of the particles; after that, the stabilized slurry can be transported to the application site where it can be pumped and finally get into contact with an aqueous medium in which the particulate hydrophilic polymers can act as a thickener.

Further subject of the present invention is a liquid composition comprising one or more liquid hydrocarbons as a carrier fluid or carrier fluid mixture, one or more insoluble solids in particulate form, and one or more urea and urethane group containing products of the present invention. A liquid hydrocarbon is a hydrocarbon or a mixture of hydrocarbons, which is flowable at 23 °C and 100 kPa. The term "insoluble solids in particulate form" means that the solubility of the particles in said liquid composition is below 25 g/l, preferably below 10 g/l, more preferably below 5 g/l at 23 °C and 100 kPa and that the particles are not liquid at 23 °C and 100 kPa. Such liquid compositions being particularly suitable in gas and oil production, preferably for drilling, stimulation and/or completion purposes.

The liquid hydrocarbons used therein are preferably selected from the group consisting of aliphatic, aromatic, or araliphatic hydrocarbons. They can be of natural origin (e.g., made of crude oil or gas) or be completely of synthetic origin. Examples are refined mineral oils, Diesel fuel, synthetic paraffins, and synthetic olefins. Besides petrochemical sources, regenerative sources can also be employed, i.e., oils can be derived from living organisms. Typical oils are known for use in drilling fluids and similar applications, and are commonly hydrotreated light distillate. The resultant product contains minimal, if any, quantities of aromatic components, and mostly short chain hydrocarbons. The LVT^{®} oil of Calumet Penrico, LLC, and the Low Toxicity Drilling Mud Oil of ExxonMobil, such as those based on ESCAIDTM fluids, are commercial examples of such products. Synthesized biodegradable oils based on alpha or internal olefins or the like are also acceptable for use as a base fluid, such as AMODRILL^{®} olefin fluid by INEOS USA, LLC, as well as ODC^{®} high purity hydrocarbons of Sasol North America, Inc., and XP-07^{®} Base from Halliburton (an example for a synthetic paraffin base oil). Furthermore, metal working fluids as well as lubricants of all API groups (I-V) are examples of hydrocarbons.

The insoluble solids in particulate form used therein are preferably selected from the group consisting of inorganic and organic particles, preferably organic particles, more preferably hydrophilic polymers, most preferably from the group of polysaccharides and even more preferred from the group consisting of cellulose ethers, such as carboxymethyl cellulose (CMC), hydroxyethyl cellulose (HEC), carboxymethyl hydroxyethyl cellulose (CMHEC) and polyanionic cellulose (PAC); guar gum, such as straight guar (Guar) and guar gum derivatives such as carboxymethyl guar (CMG), hydroxypropyl guar (HPG), and carboxymethyl hydroxyethyl guar (CMHPG); xanthan gum and xanthan gum derivatives; and starch including starch derivatives, such as carboxymethyl starch, hydroxyethyl starch, and hydroxypropyl starch.

The amount of components in the liquid composition comprising one or more liquid hydrocarbons as a carrier fluid or carrier fluid mixture, one or more insoluble solids in particulate form and the one or more urea and urethane group containing products of the present invention are preferably:
19.98 to 94.98 % by weight, preferably 29.95 to 79.95 % by weight, more preferred 34.90 to 69.90 % by weight and most preferred 39.85 to 59.85 % by weight of the one or more liquid hydrocarbons as carrier fluid, based on the total weight of the liquid composition.

5.00 to 80.00 % by weight, preferably 20.00 to 70.00 % by weight, more preferred 30.00 to 65.00 % by weight and most preferred 40.00 to 60.00 % by weight of the one or more insoluble solids in particulate form, based on the total weight of the liquid composition.

0.02 to 8.00 % by weight, preferably 0.05 to 5.00 % by weight, more preferred 0.10 to 4.00 % by weight or 0.15 to 3.00 % by weight and most preferred 0.20 to 2.00 % by weight or 0.25 to 1.50 % by weight of the one or more urea and urethane group containing products of the present invention, based on the total weight of the liquid composition.

Further object of the present invention is the use of the liquid compositions of the present invention as a rheology control additive, preferably an anti-settling agent. In particular the rheology control additive is used to control the rheology of a coating composition, a clear coat composition, a lacquer, a plastic formulation, a pigment paste, an effect pigment paste, a sealant formulation, a cosmetic formulation, a ceramic formulation, an adhesive formulation, a liquid formulation for use in gas and oil production, a composition for the manufacture of electrical components and circuits, a liquid formulation for use in energy storage media, a cleaning agent, a potting compound, a building material formulation, a lubricant, a filling compound, a wax emulsion, a metalworking fluid, a metal-processing product, a liquid composition in the form of a spraying agent, a so-called deposition aid (e.g., for use in in plant protection agents or for the general purpose of drift reduction), an ink, a printing ink and an ink jet ink.

Therefore, another object of the present invention is a process for rheology adjustment, comprising the step of adding the liquid compositions of the present invention to a coating composition, a clear coat composition, a lacquer, a plastic formulation, a pigment paste, an effect pigment paste, a sealant formulation, a cosmetic formulation, a ceramic formulation, an adhesive formulation, a liquid formulation for use in gas and oil production, a composition for the manufacture of electrical components and circuits, a liquid formulation for use in energy storage media, a cleaning agent, a potting compound, a building material formulation, a lubricant, a filling compound, a wax emulsion, a metalworking fluid, a metal-processing product, a liquid composition in the form of a spraying agent, a so-called deposition aid (e.g., for use in plant protection agents or for the general purpose of drift reduction), an ink, a printing ink and an ink jet ink.

Further liquid compositions wherein the urea group containing products of the present invention and the rheology control additives of the present invention can be used are preferably solvent-based or solvent-free paints, printing inks and inks and lacquers as e.g. lacquers for varnishing of plastics, wire enamels, coating compositions for coating foodstuffs and seeds, and as so-called color resists, which are used for color filters, for example in flat panel displays such as liquid-crystal displays. The field of application lacquers also includes pasty materials which generally have a very high proportion of solids and a small proportion of liquid components, for example so-called pigment pastes or also pastes based on effect pigments, for example metal effect pigments such as, for example, aluminum pigments, silver pigments, brass pigments, zinc pigments, copper pigments, bronze pigments such as gold bronzes, fire-dyed bronzes or iron oxide aluminum pigments. The effect pigments also include, for example, interference pigments or pearlescent pigments such as, for example, metal oxide mica pigments, fish silver, bismuth oxide chloride or basic lead carbonate.

The plastic formulations can be (liquid) starting materials to produce plastic materials, which are preferably converted into a duromer by a chemical cross-linking process ("curing"). Preferred plastic preparations are unsaturated polyester resins, vinyl ester resins, acrylate resins, epoxy resins, polyurethane resins, formaldehyde resins (such as melamine-formaldehyde or ureaformaldehyde). These can be cured under very different conditions, e.g. at room temperature (cold-curing systems) or at elevated temperature (hot-curing systems), optionally with application of pressure ("closed mold" application, sheet molding compound or bulk molding compound). The plastic formulations also include PVC plastisols.

The cosmetic preparations can be various liquid compositions, which are used in the so-called personal care or healthcare sector, e.g. lotions, creams, pastes such as, for example, toothpaste, foams such as, for example, shaving foam, gels such as, for example, shaving gels, shower gels or active ingredients in gel formulations, hair shampoos, liquid soaps, nail varnishes, lipsticks and hair dyes.

The so-called wax emulsions are preferably dispersions of solid waxes in particulate form at room temperature in water or an organic medium.

The building material formulations may be liquid or paste-like materials, which are used in the construction sector and solidify after curing. Examples are hydraulic binders such as concrete, cement, mortar, tile glue and plaster.

The metal working fluids may be cutting liquids, drilling fluids (such as are used in metal processing), or forging fluids or lubricants in general. Potential other areas are release agents (often in the form of aqueous emulsions, for example, aluminum die casting and foundry applications), foundry washes (foundry coatings) and liquids for the surface treatment of metals (for example "surface finishing", surface treatment and plating).

The lubricants and metal working fluids are means which are used for lubrication, that is to say, which serve to reduce friction and wear, as well as to provide power, cooling, vibration dampening, sealing action and corrosion protection; liquid lubricants being preferred here.

Cleaning agents can be used to clean a wide range of objects. They effect or assist the removal of impurities, residues and attachments. The cleaners also include detergents (such as for cleaning textiles, their precursors, leather, and dishes), and personal care products.

The adhesives can be all adhesive materials which are liquid under processing conditions and which can join parts by surface adhesion and internal strength.

In the above applications, the liquid composition of the invention may comprise constituents such as film-forming resins. Examples of film-forming resins are polyurethanes (1-component and 2-component systems), polyacrylates, polyester resins, alkyd resins, epoxy resins, PVC plastisols, PVC organosols, thermoplastics, and unsaturated polyester resins.

The liquid compositions of the invention may further comprise customary additives. Examples of additives are antiblocking agents, stabilizers, antioxidants, pigments, wetting agents, dispersants, emulsifiers, rheology additives, UV absorbers, free-radical scavengers, slip additives, defoamers, adhesion promoters, leveling agents, waxes, nanoparticles, film-forming auxiliaries, and flame retardants. Preferred additives are wetting agents, dispersants and/or emulsifiers and rheology additive which are different from the rheology control additives of the present invention, such as clay based thickeners (including organoclays), other urea compounds, (poly)amides, polysaccharides (like cellulose derivatives, guar, xanthan), polyacrylates, or associative thickeners. In an example, the urea group containing product of the invention can be used in combination with other thickeners affecting the low, medium, and/or high shear performance of the liquid composition that needs to be modified concerning its rheological behavior.

The urea and urethane group containing products of the invention are used in such a way that in a liquid composition, where the liquid composition is a semi-finished or final product, there is preferably 0.1 % to 10.0% by weight, more preferably 0.1 % to 8.0% by weight, and very preferably 0.2% to 5.0% by weight of the urea and urethane group containing product, based on the total weight of the liquid composition.

A further subject of the present invention is a process for exploiting a gas and oil reservoir in which a liquid composition comprising one or more urea and urethane group containing products of the present invention are used in one of the processing steps employed to make the reservoir accessible and exploit the gas and oil reserves of the reservoir.

Still a further subject of the present invention is a process for exploiting a gas and oil reservoir in which a liquid composition comprising one or more liquid hydrocarbons as a carrier fluid or carrier fluid mixture, one or more insoluble solids in particulate form, and one or more urea and urethane group containing products of the present invention are used in one of the processing steps employed to make the reservoir accessible and exploit the gas and oil reserves of the reservoir.

Preferably the process for exploiting a gas and oil deposit is selected from drilling, stimulation, completion, production, and hydraulic fracturing processes.

The invention is illustrated further below giving reference to examples.

### EXAMPLES

### SYNTHESIS EXAMPLES

**Table 1: Explanation of Abbreviations**

| *Product name* | *Chemical Composition* | *Supplier* |
|---|---|---|
| TDI T100 | 2,4-toluylene diisocyanate | Covestro AG |
| TDI T80 | 80/20 mixture of 2,4-toluylene diisocyanate and 2,6-toluylene diisocyanate | Covestro AG |
| TDI T65 | 65/35 mixture of 2,4-toluylene diisocyanate and 2,6-toluylene diisocyanate | Covestro AG |
| IPDI | isophorone diisocyanate | Merck |
| Jeffamine^{®} EDR 148 | H₂N-CH₂-CH₂-O-CH₂-CH₂-O-CH₂-CH₂-NH₂ | Huntsman Corp. |

### Manufacture of Intermediates A1 to A4:

Diisocyanates were reacted with mono alcohols according to the procedure described in EP 1188779 to form monoadducts (intermediates), containing one urethane group and one NCO group.

**Table 2: Intermediates**

| *Intermediate* | *Mono alcohol* | *Diisocyanate* |
|---|---|---|
| **A1** | (Z)-Octadec-9-enol (oleocetyl alcohol 90-95, Mosselman) | TDI T65 |
| **A2** | tridecyl alcohol (Exxal 13, Exon Mobile Chemical) | TDI T65 |
| **A3** | 2-Decyltetradecanol (Isofol 24, Sasol Performance Chemicals, Hamburg) | TDI T65 |
| **A4** | (Z)-Octadec-9-enol (oleocetyl alcohol 90-95, Mosselman) | IPDI |

### Intermediates A1 to A3:

2 mol of TDI T65 and 200 ppm benzoyl chloride were weighed into a glass flask equipped with stirrer, reflux condenser and nitrogen inlet and heated to 40 °C. Subsequently 1 mol of the mono alcohol (according to the above table 2) was added dropwise to the reaction mixture over a period of 30 min. The reaction mixture was stirred for additional 5 hours at 60 °C. A clear, light yellow, liquid crude intermediate containing excessive diisocyanate is obtained. The excessive diisocyanate contained in the crude intermediates obtained, was removed by distillation, whereby intermediates **A1** to **A3** were obtained.

### Intermediate A4:

2 mol of IPDI were weighed into a glass flask equipped with stirrer, reflux condenser and nitrogen inlet and heated to 40 °C. Subsequently 1 mol of the mono alcohol (according to the above table 2) was added dropwise to the reaction mixture over a period of 30 min. The reaction mixture was stirred for additional 2 hours at 60 °C. A clear, light yellow, liquid crude intermediate containing excessive IPDI is obtained. The excessive IPDI contained in the crude intermediate obtained, was removed by distillation, whereby intermediate A4 was obtained.

### Comparative Examples C1 to C12 (non-inventive)

Completeness of the following reactions was evaluated with wet chemical methods by determination of NCO content and amine value.

### Comparative Rheology Additive C1:

In a glass flask with stirrer, reflux condenser and nitrogen inlet, 4.800 g (0.114 mol) lithium chloride were dissolved in 130 g N-butyl butyrolactam while stirring over a period of 30 min, whereby a clear solution was obtained. Subsequently 4.600 g (0.034 mol) m-xylylene diamine (m-XDA) were added and briefly homogenized. A uniform mixture of 36.400 g (0.076 mol) of adduct A1 and 13.300 g (0.076 mol) TDI T80 was added dropwise to the reaction mixture over a period of 50 min. The reaction mixture was stirred for additional 3 hours at 80 °C. A clear, yellow, viscous product was obtained.

### Comparative Rheology Additive C2:

In a glass flask with stirrer, reflux condenser and nitrogen inlet, 5.700 g (0.135 mol) lithium chloride were dissolved in 130 g N-butyl butyrolactam while stirring over a period of 30 min, whereby a clear solution was obtained. Subsequently 9.100 g (0.067 mol) m-xylylene diamine were added and briefly homogenized. A uniform mixture of 53.300 g (0.111 mol) of adduct A1 and 1.900 g (0.011 mol) TDI T80 was added dropwise to the reaction mixture over a period of 30 min. The reaction mixture was stirred for additional 3 hours at 80 °C. A clear, yellow, liquid product was obtained.

### Comparative Rheology Additive C3:

In a glass flask with stirrer, reflux condenser and nitrogen inlet, 5.400 g (0.127 mol) lithium chloride were dissolved in 130 g N-butyl butyrolactam while stirring over a period of 30 min, whereby a clear solution was obtained. Subsequently 8.600 g (0.063 mol) m-xylylenediamine were added and briefly homogenized. A uniform mixture of 55.500 g (0.115 mol) of adduct A1 and 1.000 g (0.006 mol) TDI T80 was added dropwise to the reaction mixture over a period of 30 min. The reaction mixture was stirred for additional 3 hours at 80 °C. A clear, yellow, liquid product was obtained.

### Comparative Rheology Additive C4:

In a glass flask with stirrer, reflux condenser and nitrogen inlet, 8.900 g (0.211 mol) lithium chloride were dissolved in 292.5 g N-butyl butyrolactam while stirring over a period of 30 min, whereby a clear solution was obtained. Subsequently 28.600 g (0.211 mol) m-xyxlene diamine were added and briefly homogenized. A uniform mixture of 101.700 g (0.211 mol) of adduct A1 and 18.300 g (0.105 mol) TDI T80 was added dropwise to the reaction mixture over a period of 30 min. The reaction mixture was stirred for additional 3 hours at 80 °C. A clear, yellow, liquid product was obtained.

### Comparative Rheology Additive C5:

In a glass flask with stirrer, reflux condenser and nitrogen inlet, 3.000 g (0.071 mol) lithium chloride were dissolved in 97.5 g N-butyl butyrolactam while stirring over a period of 30 min, whereby a clear solution was obtained. Subsequently 9.600 g (0.071 mol) m-xylylene diamine were added and briefly homogenized. A uniform mixture of 33.800 g (0.071 mol) of adduct A1 and 6.200 g (0.035 mol) TDI T80 was added dropwise to the reaction mixture over a period of 30 min. The reaction mixture was stirred for additional 3 hours at 80 °C. A clear, yellow, highly viscous product was obtained.

### Comparative Rheology Additive C6:

In a glass flask with stirrer, reflux condenser and nitrogen inlet, 6.000 g (0.141 mol) lithium chloride were dissolved in 130 g N-butyl butyrolactam while stirring over a period of 30 min, whereby a clear solution was obtained. Subsequently 9.500 g (0.070 mol) m-xylylene diamine were added and briefly homogenized. A uniform mixture of 51.700 g (0.108 mol) of adduct A1 and 2.800 g (0.016 mol) TDI T80 was added dropwise to the reaction mixture over a period of 30 min. The reaction mixture was stirred for additional 3 hours at 80 °C. A clear, yellow, liquid product was obtained.

### Comparative Rheology Additive C7:

In a glass flask with stirrer, reflux condenser and nitrogen inlet, 3.000 g (0.071 mol) lithium chloride were dissolved in 97.5 g N-butyl butyrolactam while stirring over a period of 30 min, whereby a clear solution was obtained. Subsequently 9.600 g (0.071mol) m-xylylene diamine were added and briefly homogenized. A uniform mixture of 33.800 g (0.071 mol) of adduct A1 and 6.200 g (0.035 mol) TDI T80 was added dropwise to the reaction mixture over a period of 30 min. The reaction mixture was stirred for additional 3 hours at 80 °C. A clear, yellow, liquid product was obtained.

### Comparative Rheology Additive C8:

In a glass flask with stirrer, reflux condenser and nitrogen inlet, 2.200 g (0.053 mol) lithium chloride were dissolved in 63.4 g N-butyl butyrolactam while stirring over a period of 30 min, whereby a clear solution was obtained. Subsequently 3.600 g (0.026 mol) m-xylylene diamine were added and briefly homogenized. A uniform mixture of 28.100 g (0.070 mol) of adduct A4 and 0.200 g (0.035 mol) TDI T80 was added dropwise to the reaction mixture over a period of 10 min. The reaction mixture was stirred for additional 3 hours at 80 °C. A clear, yellow, liquid product was obtained.

### Comparative Rheology Additive C9:

In a glass flask with stirrer, reflux condenser and nitrogen inlet, 2.300 g (0.055 mol) lithium chloride were dissolved in 66.0 g N-butyl butyrolactam while stirring over a period of 30 min, whereby a clear solution was obtained. Subsequently 3.700 g (0.028 mol) m-xylylene diamine were added and briefly homogenized. A uniform mixture of 29.300 g (0.052 mol) of adduct A4 and 0.200 g (0.001 mol) hexamethylene diisocyanate (HDI) was added dropwise to the reaction mixture over a period of 10 min. The reaction mixture was stirred for additional 3 hours at 80 °C. A clear, yellow, liquid product was obtained.

### Comparative Rheology Additive C10:

In a glass flask with stirrer, reflux condenser and nitrogen inlet, 2.300 g (0.055 mol) lithium chloride were dissolved in 61.8 g N-butyl butyrolactam while stirring over a period of 30 min, whereby a clear solution was obtained. Subsequently 3.700 g (0.027 mol) 1,3-diaminopropane were added and briefly homogenized. A uniform mixture of 28.800 g (0.051 mol) of adduct A4 and 0.200 g (0.001 mol) TDI T80 was added dropwise to the reaction mixture over a period of 10 min. The reaction mixture was stirred for additional 3 hours at 80 °C. A clear, yellow, liquid product was obtained.

### Comparative Rheology Additive C11:

In a glass flask with stirrer, reflux condenser and nitrogen inlet, 2.400 g (0.057 mol) lithium chloride were dissolved in 65.3 g N-butyl butyrolactam while stirring over a period of 30 min, whereby a clear solution was obtained. Subsequently 2.100 g (0.029 mol) 1,3-diaminopropane were added and briefly homogenized. A uniform mixture of 30.400 g (0.054 mol) of adduct A4 and 0.200 g (0.001 mol) hexamethylene diisocyanate was added dropwise to the reaction mixture over a period of 10 min. The reaction mixture was stirred for additional 3 hours at 80 °C. A clear, yellow, liquid product was obtained.

### Comparative Rheology Additive C12:

In a glass flask with stirrer, reflux condenser and nitrogen inlet, 5.760 g (0.136 mol) lithium chloride were dissolved in 122.6 g N-butyl butyrolactam while stirring over a period of 30 min at 60 °C, whereby a clear solution was obtained. Subsequently 7.900 g (0.068 mol) hexamethylenediamine were added and briefly homogenized. Subsequently 52.360 g (0.136 mol) of adduct A2 were added dropwise to the reaction mixture over a period of 20 min. The reaction mixture was stirred for additional 3 hours at 80 °C. A clear, yellow, liquid product was obtained.

### Examples E1 to E16 (according to the invention)

Completeness of the following reactions was evaluated with wet chemical methods by determination of NCO content and the amine value.

### Rheology Additive According to the Invention E1:

In a glass flask with stirrer, reflux condenser and nitrogen inlet, 5.300 g (0.125 mol) lithium chloride were dissolved in 130 g N-butyl butyrolactam while stirring over a period of 30 min, whereby a clear solution was obtained. Subsequently 5.600 g (0.064 mol) 1,4-diaminobutane were added and briefly homogenized. A uniform mixture of 58.600 g (0.120 mol) of adduct A1 and 0.500 g (0.003 mol) TDI T80 was added dropwise to the reaction mixture over a period of 25 min. The reaction mixture was stirred for additional 4.5 hours at 80 °C. A clear, yellow, liquid product was obtained.

### Rheology Additive According to the Invention E2:

In a glass flask with stirrer, reflux condenser and nitrogen inlet, 5.200 g (0.123 mol) lithium chloride were dissolved in 130 g N-butyl butyrolactam while stirring over a period of 30 min, whereby a clear solution was obtained. Subsequently 7.200 g (0.062 mol) 1,6-diaminohexane were added and briefly homogenized. A uniform mixture of 57.100 g (0.117 mol) of adduct A1 and 0.500 g (0.003 mol) TDI T80 was added dropwise to the reaction mixture over a period of 25 min. The reaction mixture was stirred for additional 1.5 hours at 100 °C. A clear, yellow, liquid product was obtained.

### Rheology Additive According to the Invention E3:

In a glass flask with stirrer, reflux condenser and nitrogen inlet, 5.000 g (0.118 mol) lithium chloride were dissolved in 130 g N-butyl butyrolactam while stirring over a period of 30 min, whereby a clear solution was obtained. Subsequently 6.900 g (0.059 mol) 1,6-diaminobutane were added and briefly homogenized. Subsequently 58.000 g (0.119 mol) of adduct A1 was added dropwise to the reaction mixture over a period of 25 min. The reaction mixture was stirred for additional 4.5 hours at 80 °C, 2 hours at 100 °C and 1 hour at 120 °C. A clear, yellow, liquid product was obtained.

### Rheology Additive According to the Invention E4:

In a glass flask with stirrer, reflux condenser and nitrogen inlet, 5.400 g (0.127 mol) lithium chloride were dissolved in 130 g N-butyl butyrolactam while stirring over a period of 30 min, whereby a clear solution was obtained. Subsequently 5.600 g (0.064 mol) 1,4-diaminobutane were added and briefly homogenized. A uniform mixture of 58.500 g (0.121 mol) of adduct A1 and 0.500 g (0.003 mol) hexamethylene diisocyanate was added dropwise to the reaction mixture over a period of 25 min. The reaction mixture was stirred for additional 3 hours at 80 °C. A clear, yellow, liquid product was obtained.

### Rheology Additive According to the Invention E5:

In a glass flask with stirrer, reflux condenser and nitrogen inlet, 5.400 g (0.127 mol) lithium chloride were dissolved in 130 g N-butyl butyrolactam while stirring over a period of 30 min, whereby a clear solution was obtained. Subsequently 5.600 g (0.064 mol) 1,4-diaminobutane were added and briefly homogenized. A uniform mixture of 58.400 g (0.121 mol) of adduct A1 and 0.500 g (0.003 mol) isophorone diisocyanate was added dropwise to the reaction mixture over a period of 20 min. The reaction mixture was stirred for additional 3 hours at 80 °C. A clear, yellow, liquid product was obtained.

### Rheology Additive According to the Invention E6:

In a glass flask with stirrer, reflux condenser and nitrogen inlet, 5.300 g (0.125 mol) lithium chloride were dissolved in 130 g N-butyl butyrolactam while stirring over a period of 30 min, whereby a clear solution was obtained. Subsequently 7.200 g (0.062 mol) 1,6-diaminohexane were added and briefly homogenized. A uniform mixture of 57.000 g (0.118 mol) of adduct A1 and 0.500 g (0.003 mol) hexamethylene diisocyanate was added dropwise to the reaction mixture over a period of 15 min. The reaction mixture was stirred for additional 3 hours at 80 °C. A clear, yellow, liquid product was obtained.

### Rheology Additive According to the Invention E7:

In a glass flask with stirrer, reflux condenser and nitrogen inlet, 5.200 g (0.123 mol) lithium chloride were dissolved in 130 g N-butyl butyrolactam while stirring over a period of 30 min, whereby a clear solution was obtained. Subsequently 7.200 g (0.062 mol) 1,6-diaminohexane were added and briefly homogenized. A uniform mixture of 56.900 g (0.118 mol) of adduct A1 and 0.700 g (0.003 mol) isophorone diisocyanate was added dropwise to the reaction mixture over a period of 15 min. The reaction mixture was stirred for additional 3 hours at 80 °C. A clear, yellow, liquid product was obtained.

### Rheology Additive According to the Invention E8:

In a glass flask with stirrer, reflux condenser and nitrogen inlet, 3.100 g (0.073 mol) lithium chloride were dissolved in 97.5 g N-butyl butyrolactam while stirring over a period of 30 min, whereby a clear solution was obtained. Subsequently 6.400 g (0.073 mol) 1,4-diaminobutane were added and briefly homogenized. A uniform mixture of 35.000 g (0.073 mol) of adduct A1 and 8.100 g (0.036 mol) isophorone diisocyanate was added dropwise to the reaction mixture over a period of 25 min. The reaction mixture was stirred for additional 3 hours at 80 °C. A clear, yellow, liquid product was obtained.

### Rheology Additive According to the Invention E9:

In a glass flask with stirrer, reflux condenser and nitrogen inlet, 5.600 g (0.131 mol) lithium chloride were dissolved in 130 g N-butyl butyrolactam while stirring over a period of 30 min, whereby a clear solution was obtained. Subsequently 5.800 g (0.066 mol) 1,4-diaminobutane were added and briefly homogenized. A uniform mixture of 57.600 g (0.119 mol) of adduct A1 and 1.000 g (0.006 mol) hexamethylene diisocyanate was added dropwise to the reaction mixture over a period of 25 min. The reaction mixture was stirred for additional 3 hours at 80 °C. A clear, yellow, liquid product was obtained.

### Rheology Additive According to the Invention E10:

In a glass flask with stirrer, reflux condenser and nitrogen inlet, 5.500 g (0.131 mol) lithium chloride were dissolved in 130 g N-butyl butyrolactam while stirring over a period of 30 min, whereby a clear solution was obtained. Subsequently 5.800 g (0.065 mol) 1,4-diaminobutane were added and briefly homogenized. A uniform mixture of 57.400 g (0.118 mol) of adduct A1 and 1.300 g (0.006 mol) isophorone diisocyanate was added dropwise to the reaction mixture over a period of 25 min. The reaction mixture was stirred for additional 3 hours at 80 °C. A clear, yellow, liquid product was obtained.

### Rheology Additive According to the Invention E11:

In a glass flask with stirrer, reflux condenser and nitrogen inlet, 5.500 g (0.130 mol) lithium chloride were dissolved in 130 g N-butyl butyrolactam while stirring over a period of 30 min, whereby a clear solution was obtained. Subsequently 4.700 g (0.065 mol) 1,3-diaminopropane were added and briefly homogenized. A uniform mixture of 59.300 g (0.123 mol) of adduct A1 and 0.500 g (0.003 mol) hexamethylene diisocyanate was added dropwise to the reaction mixture over a period of 25 min. The reaction mixture was stirred for additional 3 hours at 80 °C. A clear, yellow, liquid product was obtained.

### Rheology Additive According to the Invention E12:

In a glass flask with stirrer, reflux condenser and nitrogen inlet, 5.100 g (0.121 mol) lithium chloride were dissolved in 130 g N-butyl butyrolactam while stirring over a period of 30 min, whereby a clear solution was obtained. Subsequently 8.900 g (0.060 mol) Jeffamine^{®} EDR 148 were added and briefly homogenized. A uniform mixture of 55.500 g (0.115 mol) of adduct A1 and 0.500 g (0.003 mol) hexamethylene diisocyanate was added dropwise to the reaction mixture over a period of 25 min. The reaction mixture was stirred for additional 3 hours at 80 °C. A clear, yellow, liquid product was obtained.

### Rheology Additive According to the Invention E13:

In a glass flask with stirrer, reflux condenser and nitrogen inlet, 5.000 g (0.117 mol) lithium chloride were dissolved in 130 g N-butyl butyrolactam while stirring over a period of 30 min, whereby a clear solution was obtained. Subsequently 8.600 g (0.058 mol) Jeffamine^{®} EDR 148 were added and briefly homogenized. Subsequently 56.400 g (0.117 mol) of adduct A1 was added dropwise to the reaction mixture over a period of 25 min. The reaction mixture was stirred for additional 3 hours at 80 °C. A clear, yellow, liquid product was obtained.

### Rheology Additive According to the Invention E14:

In a glass flask with stirrer, reflux condenser and nitrogen inlet, 5.100 g (0.121 mol) lithium chloride were dissolved in 130 g N-butyl butyrolactam while stirring over a period of 30 min, whereby a clear solution was obtained. Subsequently 8.900 g (0.060 mol) Jeffamine^{®} EDR 148 were added and briefly homogenized. A uniform mixture of 55.400 g (0.115 mol) of adduct A1 and 0.600 g (0.003 mol) isophorone diisocyanate was added dropwise to the reaction mixture over a period of 25 min. The reaction mixture was stirred for additional 3 hours at 80 °C. A clear, yellow, liquid product was obtained.

### Rheology Additive According to the Invention E15:

In a glass flask with stirrer, reflux condenser and nitrogen inlet, 23.900 g (0.563 mol) lithium chloride were dissolved in 557.1 g N-butyl butyrolactam while stirring over a period of 30 min, whereby a clear solution was obtained. Subsequently 24.800 g (0.281 mol) 1,4-diaminobutane were added and briefly homogenized. A uniform mixture of 247.00 g (0.512 mol) of adduct A1 and 4.300 g (0.026 mol) hexamethylene diisocyanate was added dropwise to the reaction mixture over a period of 25 min. The reaction mixture was stirred for additional 3 hours at 80 °C. A clear, yellow, liquid product was obtained.

### Rheology Additive According to the Invention E16:

In a glass flask with stirrer, reflux condenser and nitrogen inlet, 6.825 g lithium chloride were dissolved in 233.24 g N-butyl butyrolactam while stirring over a period of 30 min at 60 °C, whereby a clear solution was obtained. Subsequently 5.970 g (0.081 mol) 1,3-diaminopropane were added and briefly homogenized. A uniform mixture of 112.150 g (0.210 mol) of adduct A3 and 0.650 g (0.004 mol) hexamethylene diisocyanate was added dropwise to the reaction mixture over a period of 30 min. The reaction mixture was stirred for additional 3 hours at 80 °C. A clear, yellow, liquid product was obtained.

**Table 3A - Starting compounds for Producing Comparative Rheology Additives (used amounts in mol and (gram))**

| *Additives* | *H₂N-R³-NH₂* | | | *Intermediate* | | | *OCN-R⁴-NCO* | |
|---|---|---|---|---|---|---|---|---|
| | D1 | D2 | D3 | A1 | A2 | A4 | TDI T80 | HDI |
| C1 | 0.034 (4.600) | | | 0.076 (36.400) | | | 0.076 (13.300) | |
| C2 | 0.067 (9.100) | | | 0.111 (53.300) | | | 0.011 (1.900) | |
| C3 | 0.063 (8.600) | | | 0.115 (55.000) | | | 0.006 (1.000) | |
| C4 | 0.211 (28.600) | | | 0.211 (101.700) | | | 0.105 (18.300) | |
| C5 | 0.071 (9.600) | | | 0.071 (33.800) | | | 0.035 (6.200) | |
| C6 | 0.070 (9.500) | | | 0.108 (51.700) | | | 0.016 (2.800) | |
| C7 | 0.071 (9.600) | | | 0.071 (33.800) | | | 0.035 (6.200) | |
| C8 | 0.026 (3.600) | | | | | 0.070 (28.100) | 0.001 (0.200) | |
| C9 | 0.028 (3.700) | | | | | 0.052 (29.300) | | 0.001 (0.200) |
| C10 | | 0.027 (3.700) | | | | 0.051 (28.800) | 0.001 (0.200) | |
| C11 | | 0.029 (2.100) | | | | 0.054 (30.400) | | 0.001 (0.200) |
| C12 | | | 0.068 (7.900) | | 0.136 (52.360) | | | |

| | | | | | | | | |
|---|---|---|---|---|---|---|---|---|
| D1: m-XDA; D2: 1,3-diaminopropane; D3: 1,6-diaminohexane | | | | | | | | |

**Table 3B - Starting Compounds for Producing Inventive Rheology Additives (used amounts in mol and (gram))**

| *Additives* | *H₂N-R³-NH₂* | | | | *Intermediate* | | *OCN-R⁴-NCO* | | |
|---|---|---|---|---|---|---|---|---|---|
| | D2 | D3 | D5 | D6 | A1 | A3 | HDI | TDI T80 | IPDI |
| E1 | | | | 0.064 (5.600) | 0.120 (58.600) | | | 0.003 (0.500) | |
| E2 | | 0.062 (7.200) | | | 0.117 (57.100) | | | 0.003 (0.500) | |
| E3 | | 0.059 (6.900) | | | 0.119 (58.000) | | | | |
| E4 | | | | 0.064 (5.600) | 0.121 (58.500) | | 0.003 (0.500) | | |
| E5 | | | | 0.064 (5.600) | 0.121 (58.400) | | | | 0.003 (0.500) |
| E6 | | 0.062 (7.200) | | | 0.118 (57.000) | | 0.003 (0.500) | | |
| E7 | | 0.062 (7.200) | | | 0.118 (56.900) | | | | 0.003 (0.700) |
| E8 | | | | 0.073 (6.400) | 0.073 (35.000) | | | | 0.036 (8.100) |
| E9 | | | | 0.066 (5.800) | 0.119 (57.600) | | 0.006 (1.000) | | |
| E10 | | | | 0.065 (5.800) | 0.118 (57.400) | | | | 0.006 (1.300) |
| E11 | 0.065 (4.700) | | | | 0.123 (59.300) | | 0.003 (0.500) | | |
| E12 | | | 0.060 (8.900) | | 0.115 (55.500) | | 0.003 (0.500) | | |
| E13 | | | 0.058 (8.600) | | 0.117 (56.400) | | | | |
| E14 | | | 0.060 (8.900) | | 0.115 (55.400) | | | | 0.003 (0.600) |
| E15 | | | | 0.281 (24.800) | 0.512 (247.00) | | 0.026 (4.300) | | |
| E16 | 0.081 (5.970) | | | | | 0.210 (112.150) | 0.004 (0.650) | | |

| | | | | | | | | | |
|---|---|---|---|---|---|---|---|---|---|
| D2: 1,3-diaminopropane; D3: 1,6-diaminohexane; D5: Jeffamine^{®} EDR 148; D6: 1,4-diaminobutane | | | | | | | | | |

### APPLICATION EXAMPLES AND TESTING

**Table 4: Raw Materials**

| *Product name* | *Chemical Composition* | *Manufacturer* |
|---|---|---|
| XP-07 Base | Synthetic paraffin base oil | Halliburton, SIP Ltd. |
| LVT 200 | Low viscosity base oil | Deep South Chemical Inc. |
| Guar Gum 5300 | Guar Gum | Eurotech Int. Sp. Z.z.z. |
| Celpol R | Polyanionic cellulose (PAC) | CP Kelco Oil Field Group |

**Table 5: Test Systems (TS)**

| | | *Test Systems (Reference Systems without Additive) Amounts in parts by weight* | | | |
|---|---|---|---|---|---|
| | | TS1-0 | TS2-0 | TS3-0 | TS4-0 |
| Oil Component | LVT 200 | 65 | 65 | - | - |
| | XP-07 Base | - | - | 65 | 65 |
| Particle Component | Guar gum 5300 | 65 | - | 65 | - |
| | Celpol R | - | 65 | - | 65 |

### Additivation of Test Systems TS1, TS2, TS3 and TS4

The suspensions of the particle component in the oil component were prepared according to the formulations given in Table 5. 65 g of the oil component were weighed into a 250-ml glass flask and 65 g of the particle component were then added. Thereafter, the comparative and inventive rheology control additives (C1 to C12 and E1 to E16, respectively), were added as obtained in the above Experimental Section to obtain test systems containing 0.5 wt.-% and 0.25 wt.-% of the respective rheology additive based on solid ingredients (which comprises the urea and urethane group containing product and lithium chloride), respectively. The mixture was homogenized with the spatula for 1 minute. The dispersion was obtain using the shaker apparatus "Natalie" from Andalok for a period of 20 min. After shaking, a part of the homogeneous sample was transferred into 100 ml of rolled edge snap-on glasses and stored at 22 ° C for one week. The filling height in the snap-on glass was 10 cm. After one week, the homogeneity of the samples was assessed by determining the syneresis in percent of the total height. The higher the value for the syneresis, the more of the particle component deposited in the sample, i.e. the worse was the stability / homogeneity of the suspension.

The results are shown in the following Tables 6 (TS1), 7 (TS2), 8 (TS3) and 9 (TS4).

**Table 6 - Test System TS1**

| *TS1-#* | *Rheology Additive* | *Syneresis* | |
|---|---|---|---|
| | | @ dosage of 0.5 wt.-% | @ dosage of 0.25 wt.-% |
| 0 | **Reference** (no additive) | 36 | 36 |
| 1 | **C1** | 36 | - |
| 2 | **C2** | 12 | - |
| 3 | **C3** | 12 | - |
| 4 | **C4** | 16 | 18 |
| 5 | **C5** | - | 12 |
| 6 | **C6** | - | 12 |
| 7 | **C7** | - | 12 |
| 8 | **C8** | 40 | 60 |
| 9 | **C9** | 40 | 40 |
| 10 | **C10** | 40 | 35 |
| 11 | **C11** | 40 | 40 |
| 12 | **C12** | - | 15 |
| 13 | **E1** | 1 | 8 |
| 14 | **E2** | 2 | 3 |
| 15 | **E3** | 5 | 7 |
| 16 | **E4** | 0 | 4 |
| 17 | **E5** | 0 | - |
| 18 | **E6** | 2 | 4 |
| 19 | **E7** | 1 | 6 |
| 20 | **E8** | 0 | - |
| 21 | **E9** | 0 | - |
| 22 | **E10** | 0 | - |
| 23 | **E11** | 5 | - |
| 24 | **E13** | 10 | - |
| 25 | **E14** | 2 | - |
| 26 | **E15** | 5 | - |
| 27 | **E16** | 10 | - |

Some rheology control additives were tested only at the higher dosage of 0.5 wt.-% active ingredient, while other rheology control additives were only tested at the lower dosage of 0.25 wt.-%. However, some rheology control additives were also tested at both dosages.

It was surprisingly found that inventive rheology additives E1 to E11 and E13 to E16 provided a highly improved anti-settling behavior to the inventive test systems TS1-13 to TS1-27 which contain Guar Gum particles dispersed in a low viscosity base oil, compared to comparative rheology additives C1 to C12 in non-inventive test systems TS1-1 to TS1-12. In some cases, the test systems containing non-inventive rheology additives (C8 to C11) showed even worse anti-settling behavior compared to the reference test system TS1-0. Even at the lower dosage of 0.25 wt.-% of active ingredient, the inventive rheology additives performed very well.

**Table 7 - Test System TS2**

| *TS2-#* | *Rheology Additive* | *Syneresis* | |
|---|---|---|---|
| | | @ dosage 0.5 wt.-% | @ dosage 0.25 wt.-% |
| 0 | **Reference** (no additive) | 24 | 24 |
| 1 | **C1** | 31 | - |
| 2 | **C2** | 24 | - |
| 3 | **C3** | 12 | - |
| 4 | **C4** | - | 18 |
| 5 | **C5** | - | 12 |
| 6 | **C6** | - | 12 |
| 7 | **C7** | - | 12 |
| 8 | **C8** | 30 | 40 |
| 9 | **C9** | 35 | 40 |
| 10 | **C10** | 35 | 45 |
| 11 | **C11** | 35 | 40 |
| 12 | **E1** | 4 | 8 |
| 13 | **E2** | 2 | 5 |
| 14 | **E3** | 3 | 5 |
| 15 | **E4** | 0 | 1 |
| 16 | **E5** | 0 | 3 |
| 17 | **E6** | 0 | 5 |
| 18 | **E7** | 0 | 2 |
| 19 | **E8** | 5 | - |
| 20 | **E9** | 0 | 8 |
| 21 | **E10** | 5 | - |
| 22 | **E11** | 0 | 4 |
| 23 | **E12** | 0 | - |
| 24 | **E13** | 0 | 2 |
| 25 | **E14** | 0 | 2 |
| 26 | **E15** | 0 | - |

Some rheology control additives were tested only at the higher dosage of 0.5 wt.-% active ingredient, while other rheology control additives were only tested at the lower dosage of 0.25 wt.-%. However, some rheology control additives were also tested at both dosages.

It was surprisingly found that inventive rheology additives E1 to E15 provided a highly improved anti-settling behavior to the inventive test systems TS2-12 to TS2-26 which contain carboxylated cellulose particles (Celpol R) dispersed in a low viscosity base oil, compared to comparative rheology additives C1 to C11 in non-inventive test systems TS2-1 to TS2-11. In some cases, the test systems containing non-inventive rheology additives (C8 to C11) showed even worse anti-settling behavior compared to the reference test system TS2-0. Even at the lower dosage of 0.25 wt.-% of active ingredient, the inventive rheology additives performed very well.

**Table 8 - Test System TS3**

| *TS3-#* | *Rheology Additive* | *Syneresis* | |
|---|---|---|---|
| | | @ dosage 0.5 wt.-% | @ dosage 0.25 wt.-% |
| 0 | **Reference** (no additive) | 36 | 36 |
| 1 | **C1** | 40 | - |
| 2 | **C2** | 24 | - |
| 3 | **C3** | 24 | - |
| 4 | **C4** | - | 30 |
| 5 | **C5** | - | 36 |
| 6 | **C6** | - | 24 |
| 7 | **C7** | - | 36 |
| 8 | **C8** | 35 | 35 |
| 9 | **C9** | 35 | 35 |
| 10 | **C10** | 40 | 35 |
| 11 | **C11** | 35 | 35 |
| 12 | **C12** | 25 | - |
| 13 | **E1** | 3 | - |
| 14 | **E2** | 11 | 17 |
| 15 | **E3** | 10 | 20 |
| 16 | **E4** | 3 | 20 |
| 17 | **E5** | 8 | 20 |
| 18 | **E6** | 8 | 20 |
| 19 | **E7** | 10 | - |
| 20 | **E9** | 5 | - |
| 21 | **E11** | 2 | 20 |
| 22 | **E12** | 5 | 20 |
| 23 | **E13** | 15 | - |
| 24 | **E14** | 15 | - |
| 25 | **E15** | 0 | 15 |
| 26 | **E16** | 20 | - |

Some rheology control additives were tested only at the higher dosage of 0.5 wt.-% active ingredient, while other rheology control additives were only tested at the lower dosage of 0.25 wt.-%. However, some rheology control additives were also tested at both dosages.

It was surprisingly found that inventive rheology additives provided a highly improved anti-settling behavior to the inventive test systems TS3-13 to TS3-26 which contain Guar Gum particles dispersed in synthetic paraffin base oil, compared to comparative rheology additives C1 to C12 in non-inventive test systems TS3-1 to TS3-12. In most cases the test systems containing non-inventive rheology additives showed even worse anti-settling behavior compared to the reference test system TS3-0. Even at the lower dosage of 0.25 wt.-% of active ingredient, the inventive rheology additives performed well.

**Table 9 - Test System TS4**

| *TS4-#* | *Rheology Additive* | *Syneresis* | |
|---|---|---|---|
| | | @ dosage 0.5 wt.-% | @ dosage 0.25 wt.-% |
| 0 | **Reference** (no additive) | 24 | 24 |
| 1 | **C1** | 43 | - |
| 2 | **C2** | 36 | - |
| 3 | **C3** | 36 | - |
| 4 | **C4** | 26 | 33 |
| 5 | **C5** | - | 24 |
| 6 | **C6** | - | 24 |
| 7 | **C7** | - | 24 |
| 8 | **C8** | 40 | 40 |
| 9 | **C9** | 35 | 45 |
| 10 | **C10** | 45 | 40 |
| 11 | **C11** | 40 | 40 |
| 12 | **E1** | 3 | 18 |
| 13 | **E2** | 5 | 13 |
| 14 | **E3** | 4 | 10 |
| 15 | **E4** | 2 | 14 |
| 16 | **E5** | 4 | 20 |
| 17 | **E6** | 13 | 15 |
| 18 | **E7** | 3 | 14 |
| 19 | **E8** | 10 | - |
| 20 | **E9** | 5 | 15 |
| 21 | **E10** | - | 20 |
| 22 | **E11** | 0 | 15 |
| 23 | **E12** | 5 | 10 |
| 24 | **E13** | 5 | 10 |
| 25 | **E14** | 15 | 15 |
| 26 | **E15** | 5 | 20 |

Some rheology control additives were tested only at the higher dosage of 0.5 wt.-% active ingredient, while other rheology control additives were only tested at the lower dosage of 0.25 wt.-%. However, some rheology control additives were also tested at both dosages.

It was surprisingly found that inventive rheology additives provided a highly improved anti-settling behavior to the inventive test systems TS4-12 to TS3-26 which contain carboxylated cellulose particles (Celpol R) dispersed in synthetic paraffin base oil, compared to comparative rheology additives C1 to C11 in non-inventive test systems TS4-1 to TS4-11. In most cases the test systems containing non-inventive rheology additives showed even worse anti-settling behavior compared to the reference test system TS4-0. Even at the lower dosage of 0.25 wt.-% of active ingredient, the inventive rheology additives performed well.

## Claims

1. A urea and urethane group containing product comprising one or more species of formula (I)
R¹-O-(C=O)-NH-R²-NH-(C=O)-NH-R³-NH-[-(C=O)-NH-R⁴-NH-(C=O)-NH-R³-NH-]ₙ-(C=O)-NH-R²-NH-(C=O)-O-R¹ (I)
, wherein
R¹ independently represent a non-aromatic hydrocarbyl groups having 14 to 30 carbon atoms;
R² independently represent alkyl-substituted aromatic hydrocarbyl groups having 7 to 12 carbon atoms;
R³ independently represent hydrocarbyl groups having 2 to 36 carbon atoms, which can be interrupted by 1 to 17 ether oxygen atoms in case of aliphatic hydrocarbyl groups;
R⁴ independently represent hydrocarbyl groups having 2 to 36 carbon atoms;
n is an integer from 0 to 200; and
wherein on average from 40 mol-% to 100 mol-% of all R³ and R⁴ groups contained in the one or more species of formula (I) are acyclic aliphatic hydrocarbyl groups which, in case of R³, can be interrupted by 1 to 17 ether oxygen atoms.

2. The urea and urethane group containing product according to claim 1, wherein R¹ independently represents branched aliphatic hydrocarbyl groups.

3. The urea and urethane group containing product according to any one of the preceding claims, wherein R¹ independently represent ethylenically unsaturated aliphatic hydrocarbyl groups.

4. The urea and urethane group containing product according to any one of the preceding claims, wherein R¹ is selected from such aliphatic hydrocarbyl groups contained in alcohols of formula R¹-OH, where the alcohol is liquid at 23 °C (100 kPa standard pressure).

5. The urea and urethane group containing product according to any one of claims 1, 3 or 4, wherein R¹ is an octadecenyl group.

6. The urea and urethane group containing product according to any one of claims 1 to 5, wherein on average from 50 mol-% to 100 mol-%, preferably on average from 60 mol-% to 100 mol-% of all R³ and R⁴ groups contained in the one or more species of formula (I) are acyclic aliphatic hydrocarbyl groups which, in case of R³, can be interrupted by 1 to 17 ether oxygen atoms.

7. A method of manufacturing urea group containing product as defined in any one of the preceding claims, by
reacting one or more components R¹-OH with one or more diisocyanates OCN-R²-NCO to form one or more monoisocyanato adducts having the following formula (II)
R¹-O-(CO)-NH-R²-NCO (II),
and subsequently reacting a mixture of the monoisocyanato adducts having formula (II), optionally, one or more diisocyanates OCN-R⁴-NCO, and
one or more diamines H₂N-R³-NH₂,
wherein R¹, R², R³ and R⁴ are independently defined as for the species of formula (I) of claim 1.

8. A liquid composition comprising the urea and urethane group containing product according to any one of the preceding claims and a carrier medium.

9. The liquid composition according to claim 8, wherein the carrier medium is an organic solvent selected from the group consisting of amides, sulfoxides and ionic liquids.

10. The liquid composition according to claim 8 or 9, comprising or consisting of
a. 5 to 70 % by weight of one or more urea group containing products according to the invention,
b. 30 to 95 % by weight of one or more polar aprotic solvents and/or ionic liquids, and
c. 0 to 8 % by weight of one or more ionogenic compounds,
the amounts of (a), (b) and (c) being based on the total weight of the liquid composition.

11. The liquid composition according to one of claims 8 or 9 comprising particles and the particles preferably comprising or consisting of hydrophilic polymers, more preferably polysaccharides.

12. The liquid composition according to anyone of claims 8 to 11, being selected from the group consisting of a coating composition, a clear coat composition, a lacquer, a color resist, a plastic formulation, a pigment paste, an effect pigment paste, a sealant formulation, a wire enamel, a cosmetic formulation, a ceramic formulation, an adhesive formulation, a liquid formulation for use in gas and oil production, a composition for the manufacture of electrical components and circuits, a liquid formulation for use in energy storage media, a cleaning agent, a potting compound, a building material formulation, a lubricant, a filling compound, a wax emulsion, a metal-processing product, a metalworking fluid, a liquid composition in the form of a spraying agent, a deposition aid, an ink, a printing ink and an ink jet ink.

13. The liquid composition according to claim 12, wherein the liquid is for use in gas or oil production.

14. The liquid composition according to claims 12 or 13, comprising as components
one or more liquid hydrocarbons as a carrier medium,
one or more insoluble solids in particulate form; and
0.02 to 8.00 % by weight of one or more urea and urethane group containing products as claimed in any of claims 1 to 6,
based on the total weight of the liquid composition.

15. Use of the liquid composition as defined in any one of claims 8 to 10 as a rheology control additive, preferably an anti-settling agent.

16. The use according to claim 15, wherein the rheology control additive is used to control the rheology of a coating composition, a clear coat composition, a lacquer, a color resist, a plastic formulation, a pigment paste, an effect pigment paste, a sealant formulation, a wire enamel, a cosmetic formulation, a ceramic formulation, an adhesive formulation, a liquid formulation for use in gas and oil production, a liquid composition for the manufacture of electrical components and circuits, a liquid formulation for use in energy storage media, a cleaning agent, a potting compound, a building material formulation, a lubricant, a filling compound, a wax emulsion, a metal-processing product, a metalworking fluid, a liquid composition in the form of a spraying agent, a deposition aid, an ink, a printing ink and an ink jet ink.

17. A process for rheology adjustment, comprising the step of adding the liquid composition according to any of claims 8 to 10 to a coating composition, a clear coat composition, a lacquer, a color resist, a plastic formulation, a pigment paste, an effect pigment paste, a sealant formulation, a wire enamel, a cosmetic formulation, a ceramic formulation, an adhesive formulation, a liquid formulation for use in gas and oil production, a liquid composition for the manufacture of electrical components and circuits, a liquid formulation for use in energy storage media, a cleaning agent, a potting compound, a building material formulation, a lubricant, a filling compound, a wax emulsion, a metal-processing product, a metalworking fluid, a liquid composition in the form of a spraying agent, a deposition aid, an ink, a printing ink and an ink jet ink.

## Patentansprüche

1. Harnstoff- und Urethangruppen enthaltendes Produkt, umfassend eine oder mehrere Spezies der Formel (I)
R¹-O-(C=O)-NH-R²-NH-(C=O)-NH-R³-NH-[-(C=O)-NH-R⁴-NH-(C=O)-NH-R³-NH-]ₙ-(C=O)-NH-R²-NH-(C=O)-R¹ (I)
, wobei
R¹ unabhängig für nichtaromatische Hydrocarbylgruppen mit 14 bis 30 Kohlenstoffatomen steht;
R² unabhängig für alkylsubstituierte aromatische Hydrocarbylgruppen mit 7 bis 12 Kohlenstoffatomen steht;
R³ unabhängig für nichtaromatische Hydrocarbylgruppen mit 2 bis 36 Kohlenstoffatomen steht, die im Fall von aliphatischen Hydrocarbylgruppen durch 1 bis 17 Ether-Sauerstoffatome unterbrochen sein können;
R⁴ unabhängig für Hydrocarbylgruppen mit 2 bis 36 Kohlenstoffatomen steht;
n eine ganze Zahl von 0 bis 200 ist und
wobei durchschnittlich 40 Mol-% bis 100 Mol-% aller R³- und R⁴-Gruppen, die in der einen bzw. den mehreren Spezies der Formel (I) enthalten sind, acyclische aliphatische Hydrocarbylgruppen sind, die im Fall von R³ durch 1 bis 17 Ether-Sauerstoffatome unterbrochen sein können.

2. Harnstoff- und Urethangruppen enthaltendes Produkt nach Anspruch 1, wobei R¹ unabhängig für verzweigte aliphatische Hydrocarbylgruppen steht.

3. Harnstoff- und Urethangruppen enthaltendes Produkt nach einem der vorhergehenden Ansprüche, wobei R¹ unabhängig für ethylenisch ungesättigte aliphatische Hydrocarbylgruppen steht.

4. Harnstoff- und Urethangruppen enthaltendes Produkt nach einem der vorhergehenden Ansprüche, wobei R¹ aus solchen aliphatischen Hydrocarbylgruppen ausgewählt ist, die in Alkoholen der Formel R¹-OH enthalten sind, wobei der Alkohol bei 23 °C (100 kPa Normaldruck) flüssig ist.

5. Harnstoff- und Urethangruppen enthaltendes Produkt nach einem der Ansprüche 1, 3 oder 4, wobei R¹ eine Octadecenylgruppe ist.

6. Harnstoff- und Urethangruppen enthaltendes Produkt nach einem der Ansprüche 1 bis 5, wobei durchschnittlich 50 Mol-% bis 100 Mol-%, bevorzugt durchschnittlich 60 Mol-% bis 100 Mol-%, aller R³- und R⁴-Gruppen, die in der einen bzw. den mehreren Spezies der Formel (I) enthalten sind, acyclische aliphatische Hydrocarbylgruppen sind, die im Fall von R³ durch 1 bis 17 Ether-Sauerstoffatome unterbrochen sein können.

7. Verfahren zur Herstellung eines Harnstoffgruppen enthaltenden Produkts gemäß einem der vorhergehenden Ansprüche durch
Umsetzung einer oder mehrerer Komponenten R¹-OH mit einem oder mehreren Diisocyanaten OCN-R²-NCO zur Bildung eines oder mehrerer Monoisocyanato-Addukte der folgenden Formel (II)
R¹-O-(CO)-NH-R²-NCO (II)
und anschließende Umsetzung einer Mischung der Monoisocyanato-Addukte der Formel (II), gegebenenfalls eines oder mehreren Diisocyanate OCN-R⁴-NCO und eines oder mehrerer Diamine H₂N-R³-NH₂,
wobei R¹, R², R³ und R⁴ unabhängig wie für die Spezies der Formel (I) aus Anspruch 1 definiert sind.

8. Flüssige Zusammensetzung, umfassend das Harnstoff- und Urethangruppen enthaltende Produkt nach einem der vorhergehenden Ansprüche und ein Trägermedium.

9. Flüssige Zusammensetzung nach Anspruch 8, wobei es sich bei dem Trägermedium um ein organisches Lösungsmittel aus der Gruppe bestehend aus Amiden, Sulfoxiden und ionischen Flüssigkeiten handelt.

10. Flüssige Zusammensetzung nach Anspruch 8 oder 9, umfassend oder bestehend aus
a. 5 bis 70 Gew.-% eines oder mehrerer erfindungsgemäßer Harnstoffgruppen enthaltender Produkte,
b. 30 bis 95 Gew.-% eines oder mehrerer polarer aprotischer Lösungsmittel und/oder einer oder mehrerer ionischer Flüssigkeiten und
c. 0 bis 8 Gew.-% einer oder mehrerer ionogener Verbindungen,
wobei sich die Mengen von (a), b) und (c) auf das Gesamtgewicht der flüssigen Zusammensetzung beziehen.

11. Flüssige Zusammensetzung nach Anspruch 8 oder 9, die Teilchen umfasst, wobei die Teilchen vorzugsweise hydrophile Polymere, weiter bevorzugt Polysaccharide, umfassen oder daraus bestehen.

12. Flüssige Zusammensetzung nach einem der Ansprüche 8 bis 11, die ausgewählt ist aus der Gruppe bestehend aus einer Beschichtungszusammensetzung, einer Klarlackzusammensetzung, einem Lack, einem Color Resist, einer Kunststoffformulierung, einer Pigmentpaste, einer Effektpigmentpaste, einer Dichtstoffformulierung, einem Drahtlack, einer Kosmetikformulierung, einer Keramikformulierung, einer Klebstoffformulierung, einer flüssigen Formulierung zur Verwendung bei der Gas- und Ölförderung, einer Zusammensetzung zur Herstellung von elektrischen Bauteilen und Schaltungen, einer flüssigen Formulierung zur Verwendung in Energiespeichermedien, einem Reinigungsmittel, einer Vergussmasse, einer Baustoffformulierung, einem Schmiermittel, einer Spachtelmasse, einer Wachsemulsion, einem Metallverarbeitungsprodukt, einer Metallbearbeitungsflüssigkeit, einer flüssigen Zusammensetzung in Form eines Sprühmittels, einem Deposition Aid, einer Tinte, einer Druckfarbe und einer Tintenstrahltinte.

13. Flüssige Zusammensetzung nach Anspruch 12, wobei die Flüssigkeit zur Verwendung bei der Gas- oder Ölförderung vorgesehen ist.

14. Flüssige Zusammensetzung nach Anspruch 12 oder 13, umfassend als Komponenten
einen oder mehrere flüssige Kohlenwasserstoffe als Trägermedium,
einen oder mehrere unlösliche Feststoffe in Teilchenform und
0,02 bis 8,00 Gew.-% eines oder mehrerer Harnstoff- und Urethangruppen enthaltender Produkte nach einem der Ansprüche 1 bis 6,
bezogen auf das Gesamtgewicht der flüssigen Zusammensetzung.

15. Verwendung der flüssigen Zusammensetzung gemäß einem der Ansprüche 8 bis 10 als Rheologiesteuerungsmittel, vorzugsweise als Antiabsetzmittel.

16. Verwendung nach Anspruch 15, wobei das Rheologiesteuerungsmittel zur Steuerung der Rheologie einer Beschichtungszusammensetzung, einer Klarlackzusammensetzung, eines Lacks, eines Color Resist, einer Kunststoffformulierung, einer Pigmentpaste, einer Effektpigmentpaste, einer Dichtstoffformulierung, eines Drahtlacks, einer Kosmetikformulierung, einer Keramikformulierung, einer Klebstoffformulierung, einer flüssigen Formulierung zur Verwendung bei der Gas-und Ölförderung, einer flüssigen Zusammensetzung zur Herstellung von elektrischen Bauteilen und Schaltungen, einer flüssigen Formulierung zur Verwendung in Energiespeichermedien, eines Reinigungsmittels, einer Vergussmasse, einer Baustoffformulierung, eines Schmiermittels, einer Spachtelmasse, einer Wachsemulsion, eines Metallverarbeitungsprodukts, einer Metallbearbeitungsflüssigkeit, einer flüssigen Zusammensetzung in Form eines Sprühmittels, eines Deposition Aid, einer Tinte, einer Druckfarbe und einer Tintenstrahltinte verwendet wird.

17. Verfahren zur Rheologieeinstellung, umfassend den Schritt des Zugebens der flüssigen Zusammensetzung nach einem der Ansprüche 8 bis 10 zu einer Beschichtungszusammensetzung, einer Klarlackzusammensetzung, einem Lack, einem Color Resist, einer Kunststoffformulierung, einer Pigmentpaste, einer Effektpigmentpaste, einer Dichtstoffformulierung, einem Drahtlack, einer Kosmetikformulierung, einer Keramikformulierung, einer Klebstoffformulierung, einer flüssigen Formulierung zur Verwendung bei der Gas- und Ölförderung, einer flüssigen Zusammensetzung zur Herstellung von elektrischen Bauteilen und Schaltungen, einer flüssigen Formulierung zur Verwendung in Energiespeichermedien, einem Reinigungsmittel, einer Vergussmasse, einer Baustoffformulierung, einem Schmiermittel, einer Spachtelmasse, einer Wachsemulsion, einem Metallverarbeitungsprodukt, einer Metallbearbeitungsflüssigkeit, einer flüssigen Zusammensetzung in Form eines Sprühmittels, einem Deposition Aid, einer Tinte, einer Druckfarbe und einer Tintenstrahltinte.

## Revendications

1. Produit contenant un groupe urée et uréthane comprenant une ou plusieurs espèces de formule (I)
R¹-O-(C=O)-NH-R²-NH-(C=O)-NH-R³-NH-[-(C=O)-NH-R⁴-NH-(C=O)-NH-R³-NH-]ₙ-(C=O)-NH-R²-NH-(C=O)-R¹ (I)
,
R¹ représentant indépendamment des groupes hydrocarbyle non aromatiques possédant 14 à 30 atomes de carbone ;
R² représentant indépendamment des groupes hydrocarbyle aromatiques, possédant 7 à 12 atomes de carbone, substitués par alkyle ;
R³ représentant indépendamment des groupes hydrocarbyle possédant 2 à 36 atomes de carbone, qui peuvent être interrompus par 1 à 17 atomes d'oxygène d'éther dans le cas de groupes hydrocarbyle aliphatiques ;
R⁴ représentant indépendamment des groupes hydrocarbyle possédant 2 à 36 atomes de carbone ;
n étant un entier de 0 à 200 ; et
en moyenne de 40 % en moles à 100 % en moles de tous les groupes R³ et R⁴ contenus dans l'espèce ou les espèces de formule (I) étant des groupes hydrocarbyle aliphatiques acycliques qui, dans le cas de R³, peuvent être interrompus par 1 à 17 atomes d'oxygène d'éther.

2. Produit contenant un groupe urée et uréthane selon la revendication 1, R¹ représentant indépendamment des groupes hydrocarbyle aliphatiques ramifiés.

3. Produit contenant un groupe urée et uréthane selon l'une quelconque des revendications précédentes, R¹ représentant indépendamment des groupes hydrocarbyle aliphatiques éthyléniquement insaturés.

4. Produit contenant un groupe urée et uréthane selon l'une quelconque des revendications précédentes, R¹ étant choisi parmi de tels groupes hydrocarbyle aliphatiques contenus dans des alcools de formule R¹-OH, où l'alcool est liquide à 23 °C (100 kPa de pression standard).

5. Produit contenant un groupe urée et uréthane selon l'une quelconque des revendications 1, 3 et 4, R¹ étant un groupe octadécényle.

6. Produit contenant un groupe urée et uréthane selon l'une quelconque des revendications 1 à 5, en moyenne de 50 % en moles à 100 % en moles, préférablement en moyenne de 60 % en moles à 100 % en moles de tous les groupes R³ et R⁴ contenus dans l'espèce ou les espèces de formule (I) étant des groupes hydrocarbyle aliphatiques acycliques qui, dans le cas de R³, peuvent être interrompus par 1 à 17 atomes d'oxygène d'éther.

7. Procédé de fabrication d'un produit contenant un groupe urée tel que défini dans l'une quelconque des revendications précédentes, par
mise en réaction d'un ou plusieurs composants R¹-OH avec un ou plusieurs diisocyanates OCN-R²-NCO pour former un ou plusieurs adduits de type monoisocyanato possédant la formule suivante (II)
R¹-O-(CO)-NH-R²-NCO (II),
et subséquemment mise en réaction d'un mélange des adduits de type monoisocyanato possédant la formule (II), éventuellement, d'un ou plusieurs diisocyanates OCN-R⁴-NCO, et
d'une ou plusieurs diamines H₂N-R³-NH₂,
R¹, R², R³ et R⁴ étant indépendamment définis comme pour les espèces de formule (I) selon la revendication 1.

8. Composition liquide comprenant le produit contenant un groupe urée et uréthane selon l'une quelconque des revendications précédentes et un milieu de support.

9. Composition liquide selon la revendication 8, le milieu de support étant un solvant organique choisi dans le groupe constitué par des amides, des sulfoxydes et des liquides ioniques.

10. Composition liquide selon la revendication 8 ou 9, comprenant ou constituée de
a. 5 à 70 % en poids d'un ou plusieurs produits contenant un groupe urée selon l'invention,
b. 30 à 95 % en poids d'un ou plusieurs solvants aprotiques polaires et/ou liquides ioniques, et
c. 0 à 8 % en poids d'un ou plusieurs composés ionogènes,
les quantités de (a), (b) et (c) étant basées sur le poids total de la composition liquide.

11. Composition liquide selon l'une des revendications 8 et 9 comprenant des particules et les particules préférablement comprenant ou étant constituées de polymères hydrophiles, plus préférablement de polysaccharides.

12. Composition liquide selon l'une quelconque des revendications 8 à 11, qui est choisie dans le groupe constitué par une composition de revêtement, une composition de revêtement transparent, une laque, un résist couleur, une formulation de plastique, une pâte de pigment, une pâte de pigment à effet, une formulation d'agent d'étanchéité, un vernis pour fil, une formulation cosmétique, une formulation de céramique, une formulation d'adhésif, une formulation liquide pour une utilisation dans la production de gaz et de pétrole, une composition pour la fabrication de composants et de circuits électriques, une formulation liquide pour une utilisation dans des supports de stockage d'énergie, un agent de nettoyage, un composé d'empotage, une formulation de matériau de construction, un lubrifiant, un composé de remplissage, une émulsion de cire, un produit de traitement de métal, un fluide de travail de métal, une composition liquide sous la forme d'un agent de pulvérisation, un auxiliaire de dépôt, une encre, une encre d'impression et une encre pour jet d'encre.

13. Composition liquide selon la revendication 12, le liquide étant pour une utilisation dans la production de gaz ou de pétrole.

14. Composition liquide selon la revendication 12 ou 13, comprenant en tant que composants
un ou plusieurs hydrocarbures liquides en tant que milieu de support,
un ou plusieurs solides insolubles sous forme particulaire ; et
0,02 à 8,00 % en poids d'un ou plusieurs produits contenant un groupe urée et uréthane selon l'une quelconque des revendications 1 à 6,
sur la base du poids total de la composition liquide.

15. Utilisation de la composition liquide telle que définie dans l'une quelconque des revendications 8 à 10 en tant qu'additif de régulation de la rhéologie, préférablement un agent anti-dépôt.

16. Utilisation selon la revendication 15, l'additif de régulation de la rhéologie étant utilisé pour réguler la rhéologie d'une composition de revêtement, d'une composition de revêtement transparent, d'une laque, d'un résist couleur, d'une formulation de plastique, d'une pâte de pigment, d'une pâte de pigment à effet, d'une formulation d'agent d'étanchéité, d'un vernis pour fil, d'une formulation cosmétique, d'une formulation de céramique, d'une formulation d'adhésif, d'une formulation liquide pour une utilisation dans la production de gaz et de pétrole, d'une composition liquide pour la fabrication de composants et de circuits électriques, d'une formulation liquide pour une utilisation dans des supports de stockage d'énergie, d'un agent de nettoyage, d'un composé d'empotage, d'une formulation de matériau de construction, d'un lubrifiant, d'un composé de remplissage, d'une émulsion de cire, d'un produit de traitement de métal, d'un fluide de travail de métal, d'une composition liquide sous la forme d'un agent de pulvérisation, d'un auxiliaire de dépôt, d'une encre, d'une encre d'impression et d'une encre pour jet d'encre.

17. Procédé pour l'ajustement de la rhéologie, comprenant l'étape d'ajout de la composition liquide selon l'une quelconque des revendications 8 à 10 à une composition de revêtement, une composition de revêtement transparent, une laque, un résist couleur, une formulation de plastique, une pâte de pigment, une pâte de pigment à effet, une formulation d'agent d'étanchéité, un vernis pour fil, une formulation cosmétique, une formulation de céramique, une formulation d'adhésif, une formulation liquide pour une utilisation dans la production de gaz et de pétrole, une composition liquide pour la fabrication de composants et de circuits électriques, une formulation liquide pour une utilisation dans des supports de stockage d'énergie, un agent de nettoyage, un composé d'empotage, une formulation de matériau de construction, un lubrifiant, un composé de remplissage, une émulsion de cire, un produit de traitement de métal, un fluide de travail de métal, une composition liquide sous la forme d'un agent de pulvérisation, un auxiliaire de dépôt, une encre, une encre d'impression et une encre pour jet d'encre.
